# EUROPEAN PATENT SPECIFICATION

(11) **EP 2 310 515 B1**
(45) Date of publication and mention of the grant of the patent: **16.04.2014**
(21) Application number: 09797438.0
(22) Date of filing: 15.07.2009
(51) Int. Cl.: C12N 15/82, C12N 15/12, C07K 14/435, A01H 5/00

(54) **Method to obtain transgenic plants resistant to phytopathogens based on RNA interference (RNAi)**
Verfahren zur Gewinnung von gegen Pflanzenkrankheitserregern resistenten transgenen Pflanzen auf Basis von RNA-Interferenz (RNAi)
Procédé pour obtenir des plantes transgéniques résistantes aux phytopathogènes basé sur l'interférence d'ARN (ARNi)

(30) Priority: 17.07.2008 IT MI20081305
(43) Date of publication of application: 20.04.2011
(73) Proprietor: Arterra Bioscience S.R.L., 80142 Napoli (IT)
(72) Inventor: ARCIELLO, Stefania, I-80046 San Giorgio a Cremano (IT); ANDRENACCI, Davide, I-80124 Napoli (IT); APONE, Fabio, I-80128 Napoli (IT); COLUCCI, Maria, Gabriella, I-80078 Pozzuoli (IT)
(74) Representative: Finetti, Claudia
(86) International application number: PCT/EP2009/005203
(87) International publication number: WO 2010/006804

(56) References cited:
- WO-A-03/052078
- WO-A-2006/047495
- WO-A-2006/101854
- WO-A-2008/067759
- WO-A-2008/103643
- SECHER THOMAS ET AL: "Molecular cloning of a functional allatostatin gut/brain receptor and an allatostatin preprohormone from the silkworm Bombyx mori" JOURNAL OF BIOLOGICAL CHEMISTRY, vol. 276, no. 50, 14 December 2001 (2001-12-14), pages 47052-47060, XP002524262 ISSN: 0021-9258
- NIU QI-WEN ET AL: "Expression of artificial microRNAs in transgenic Arabidopsis thaliana confers virus resistance" NATURE BIOTECHNOLOGY, NATURE PUBLISHING GROUP, NEW YORK, NY, US, vol. 24, no. 11, 1 November 2006 (2006-11-01), pages 1420-1428, XP002514067 ISSN: 1087-0156
- AMIN SADEGHI ET AL: "Ectopically expressed leaf and bulb lectins from garlic (Allium sativum L.) protect transgenic tobacco plants against cotton leafworm (Spodoptera littoralis)" TRANSGENIC RESEARCH, KLUWER ACADEMIC PUBLISHERS-PLENUM PUBLISHERS, NE, vol. 17, no. 1, 31 January 2007 (2007-01-31), pages 9-18, XP019581523 ISSN: 1573-9368
- PRICE D R G ET AL: "RNAi-mediated crop protection against insects" TRENDS IN BIOTECHNOLOGY, ELSEVIER PUBLICATIONS, CAMBRIDGE, GB, vol. 26, no. 7, 1 July 2008 (2008-07-01), pages 393-400, XP022757296 ISSN: 0167-7799
- BAUM J A ET AL: "Control of coleopteran insect pests through RNA interference" NATURE BIOTECHNOLOGY NOVEMBER 2007 NATURE PUBLISHING GROUP US, vol. 25, no. 11, November 2007 (2007-11), pages 1322-1326, XP002524149
- KEATING C D ET AL: "Whole-Genome Analysis of 60 G Protein-Coupled Receptors in Caenorhabditis elegans by Gene Knockout with RNAi" CURRENT BIOLOGY, CURRENT SCIENCE, GB, vol. 13, no. 19, 30 September 2003 (2003-09-30), pages 1715-1720, XP004545246 ISSN: 0960-9822
- BOUTROS M ET AL: "Genome-wide RNAi analysis of growth and viability in Drosophila cells" SCIENCE, AMERICAN ASSOCIATION FOR THE ADVANCEMENT OF SCIENCE, US, WASHINGTON, DC, vol. 303, no. 5659, 6 February 2004 (2004-02-06), pages 832-835, XP002352500 ISSN: 0036-8075
- DATABASE EMBL [Online] 22 December 2005 (2005-12-22), "Meloidogyne incognita flp-18 GPCR receptor (FLP-18REC) mRNA, complete cds." XP002548474 retrieved from EBI accession no. EMBL:AY882441 Database accession no. AY882441

## Description

The present invention relates to a method to obtain transgenic plants resistant to the attack of phytopathogens based on RNA interference (RNAi) for the protection of the same from the attack of parasites and phytophages, which contemplates the expression of dsRNA in the plant tissues suitable for inhibiting the functionality of a GPCR receptor, whose functioning is vital for fungi, herbivorous insects or phytopathogenic nematodes. In particular, the present invention relates to plants expressing dsRNA in their tissues, obtainable with said preventive protection treatment from the attacks of fungi, herbivorous insects or phytopathogenic nematodes.

RNAi is a natural process preserved during evolution and present in all organisms. It is a gene silencing mechanism whereby various double-stranded RNA fragments are capable of interfering and extinguishing the gene expression. Once a double-stranded RNA molecule (dsRNA) has been defined, the so-called RNAi machinery is capable of activating the RNAi mechanism (Brandt, 2002).

Through an enzyme called dicer, the dsRNA sequence is cut into fragments having a shorter length (19-21 pair of bases) (Hamilton and Baulcombe, 1999). The short dsRNA fragment (called short interfering RNA, or siRNA) is associated with an enzymatic complex called RISC (RNA-*interference silencing complex).* The dsRNA is opened, probably by means of a helicase: only the antisense RNA strand remains associated with the RISC, whereas the sense strand is degraded. The RISC complex is capable of recognizing among the numerous mRNAs present in the cytosol, which is complementary to the antisense RNA fragment associated with the same complex. If the pairing between siRNA and mRNA is perfect (or almost perfect), a RISC component (called argonaute protein) is capable of effecting a cut on the mRNA (Hammond *et al*., 2001). The two resulting mRNA fragments, one without a head at 5' and the other without a tail of A poles at 3', are therefore rapidly degraded by the RNAse of the cell itself. Another common protein, although not universally present in the RNAi machinery, is RNA dependent RNA polymerase (RdRP) which synthesizes dsRNA starting from a single helix RNA mould to produce the RNAi amplification mechanism.

The discovery of RNAi as a powerful and easy gene silencing method has awakened the attention of the whole scientific community. The ubiquity of the phenomenon has allowed it to be studied on numerous species. Many experiments are in fact based on the simple immersion of entire organisms in solutions containing dsRNA or through feeding with bacteria expressing dsRNA. This has enabled the rapid identification of genes involved in the RNAi in *C. elegans,* and discovery of their homologues in *Drosophila,* plants and fungi, and has demonstrated that phenomena, which were first classified as quelling PTGS (post-transcriptional gene silencing), are all part of a single process whose roots are founded in a single ancestral mechanism.

In the plant context, applications of gene silencing by means of RNAi have allowed varieties of agricultural interest to be produced with increased resistance levels to diseases, insects, or with high nutritional quality.

Some researchers, for example, have contributed to improve rice plants using the RNAi technique. They have reduced the levels of glutenin producing a variety of rice called *LGC-1* (low glutenin content 1) suitable for patients with renal deficiencies incapable of digesting glutenin (Kusaba *et al.,* 2003).

Other RNAi applications concerned the removal of plant endotoxins by the silencing of genes involved in the biosynthesis of the toxin. The theobromine synthase enzyme of coffee plants was inactivated with dsRNA allowing the production of decaffeinated coffee (Ogita *et al*., 2003). Silencing experiments using dsRNAs have also been carried out on pathogens and phytophages of plants of agricultural interest to inhibit the expression of some of their vital genes. Micro-injections of dsRNA in end phase larvae and adult coleopterans (*Tribolium castaneum*) have allowed genes to be silenced and their function to be studied (Tomoyasu and Denell, 2004). Some researchers have carried out tests with larvae of *Diabrotica virgifera virgifera* LeConte fed with a synthetic diet enriched with specific dsRNA to identify genes essential for the vitality of the insect. These tests led to the identification of 14 key genes whose inactivation due to interference causes larval death. Transgenic corn plants expressing one of these dsRNAs have shown high protection levels, comparable to those obtained with transgenic plants expressing the toxin *Bt* (Baum *et al*., 2007). Unlike the latter however, transgenic plants expressing dsRNA have a reduced environmental impact. The inhibition effect of insect genes, in fact, in this case depend exclusively on the specific recognition between the siRNA and target sequence to be inactivated. Problems linked to the introduction of "foreign" molecules into the environment and interactions with non-target organisms are therefore eliminated.

For the control of pathogen agents and phytophage organisms the selection of the gene target to be inactivated is of fundamental importance.

G protein coupled receptors (GPCRs) comprise a vast group of proteins, structurally and functionally similar to each other, which exert functions of vital importance in all eukaryotic organisms. They consist of a single polypeptide chain which crosses the membrane seven times with the extracellular amino-terminal end and the intracellular carboxy-terminal end; following the binding with extracellular ligands (peptides, small molecules, ions, light and aromatic compounds) the receptor is activated by triggering a response in the cell which leads to the production of a secondary message (cAMP, Ca²⁺, cGMP, IP₃).

Most organisms of agricultural interest have numerous GPCRs, the majority of which are fundamental for the correct exertion of their vital functions or for their pathogenicity. The most widely-studied species, whose genome is completely sequenced and registered and in which there is considerable information on the developmental biology, are obviously the model organisms: *Drosophila melanogaster, Caenorhabditis elegans*, *Saccharomyces* e *Neurospora. D*. *melanogaster* is the most widely-studied arthropod and is considered the model organism for the study of harmful insects. In *Drosophila* genome about 270 GPCRs have been identified, grouped into 5 families. The genome sequence of the mosquito *Anopheles gambiae* has also recently been completed. A detailed bio-informatic approach has led to the identification of 276 GPCRs in the genome of this dipteran of great sanitary interest. A great deal of information is also available on the expression specificity of these genes in the different development phases and a detailed comparative analysis has been effected with the genome of *Drosophila melanogaster.*

*C. elegans* is the model metazoan widely studied from a genetic, neurobiological, cellular and molecular point of view. Also for this organism the genome sequence is complete and a great deal of information concerning bio-informatics, expression, in addition to signal transduction, neurobiology, development, behaviour, reproduction is provided in literature. Projects financed by public institutions are also in an advanced phase, which are aimed at the identification of abut 315.000 ESTs (Expressed Sequence Tags) from 20 different species of nematode parasites. The availability of all this information will allow to clone the cDNAs of the GPCRs very rapidly and thus use them as targets of RNAis and potential nematocides.

Less information, on the other hand, is available for the GPCRs of fungi. A total of two transduction pathways mediated by GPCRs have been identified in both ascomycetes and basidiomycetes:
a) pheromone response (GPCRs = STE);
b) response to nutritional factors (glucose sensors).

Much more information is available on the heterotrimeric G proteins, direct effectors of GPCRs, in both ascomycetes and basidiomycetes (Li et al, 2007). At least 3 Gα subunits have been identified and null-mutants of various fungus species have been obtained (*Saccharomyces, Neurospora, Aspergillus, Ustilago, Fusarium, Colletotrichus, Cryphonectria, etc*.) which suggested the involvement of G proteins in a wide range of signaling pathways: pheromone response, sensitivity to nutrients, sterility, life growth, virulence and pathogenicity, development of the ascospores, morphogenesis, light. It is therefore evident that inactivating these GPCRs interferes with signals of vital importance of pathogen fungi.

In conclusion, interference by means of RNAi on the GPCRs of any organism would therefore be extremely specific as the percentage of gene homology among the GPCRs of insects, nematodes, fungi and mammals never exceeds 20-30% (see Table 1 below). Similarly, the percentage relating to the homology among the GPCRs of species belonging to the same order is never complete (around 90%) and this ensures discrimination between species of harmful organisms and those benevolent to the environment (data not published).

**Table 1. Example of homology degrees between a given insect species and other species of organisms:**

| |
|---|
| human being ≅ 20 - 30% |
| insect belonging to the same order ≅ 90% |
| insect belonging to a different order ≅ 30-55%. |

So far, most of the substances used as pesticides, nematocides or fungicides are molecules with a wide spectrum, there is therefore an enormous request for more selective methods which specifically interfere with the survival and fertility of harmful organisms, but at the same time have a minimum or no effect on non-target organisms and on the environment. The nematocides currently present on the market, for example, are highly toxic, costly and difficult to use. Methylene bromide which is the most widely-used product for the control of nematodes, fungi and bacteria on fruit trees and vegetables in California and Florida, is a neuro-toxin which also reduces the ozone in the atmosphere. Other nematocides used are aldicarb (Temik) and 1'1,3-dichloropropene (TeloneII), both carcinogenic for human beings.

On the basis of what is specified above, there is an evident demand for new methods and relative biological targets which specifically interfere with the survival and fertility of phytopathogen organisms (i.e. parasites and phytophages) more selectively and consequently having a lower environmental impact.

The Applicant has now found that GPCR receptors can be convenient targets for the control of parasites and phytophages of plants, such as fungi, insects and nematodes. Many of these GPCRs, in fact, have a gene sequence which is very specific for harmful organisms, consequently molecules of dsRNA are capable of inhibiting their functionality and cannot damage the host organism (plant) or a non-target consumer of the same plant (i.e. human being, other animals). In particular, the Applicants have prepared a method which uses the expression of dsRNA for inhibiting the functionality of a GPCR receptor, whose functioning is vital for fungi, herbivorous insects or phytopathogen nematodes.

The advantages which can be associated with the technology, object of the present invention, are summarized hereunder:
a) the selectivity, which can harm a herbivorous insect, nematode or parasitic fungus but not other benevolent organisms (i.e. bees);
b) food safety, for the same reason as above, but also for the fact that an RNA molecule can be easily degraded and does not persist in the environment;
c) the possibility of expressing dsRNA also in portions of the plant which would normally be particularly difficult to reach even with systemic chemical treatment (e.g. pyramid);
d) the potential absence of allergenic risk, as new proteins of the host plant are not expressed;
e) the possibility of selecting plants with a high/total proteomic homology with wild-type with a suitable selection of clones.

The Applicant has now found a method for obtaining transgenic plants resistant to the attack of one or more phytopathogens like Spodoptera littoralis and/or meloidogyne incognita by RNA interference comprising the following phases:
a) isolation of the cDNA nucleotide sequence encoding for a G protein coupled receptor (GPCR), or a portion thereof, whose function is vital for the phytopathogen wherein said cDNA nucleotide sequence encodes for a GPCR receptor selected between AlstCR of SEQ ID NO:2, DHR of SEQ ID NO:3 or Oct/TyrR of SEQ ID NO:6 when said insect is *Spodoptera littoralis* and wherein said cDNA nucleotide sequence encodes for a GPCR receptor selected between serotonin-like receptor of SEQ ID NO: 9 or neuropeptide receptor of SEQ ID NO:11 when said phytopathogen is *Meloidogyne incognita;*
b) construction of an expression vector comprising the cDNA nucleotide sequence encoding for a GPCR or a portion thereof as determined by step a), flanked by two specific recombination sites;
c) recombination reaction of the expression vector of step b) with a binary vector comprising a constitutive or tissue-specific promoter, the same specific recombination sites and an intronic sequence to obtain a recombinant plasmid;
d) transfer of said recombinant plasmid into competent cells of *Agrobacterium tumefaciens* and transformation of the plant;
e) growing the plant and obtaining the constitutive or tissue-specific expression of dsRNA in said plant.

The method described herein uses the double-stranded RNA expression for inhibiting the functionality of a GPCR receptor, whose functioning is vital for fungi, herbivorous insects or phytopathogen nematodes, and said dsRNA is expressed in the plant tissues so that it can be absorbed through the fungal haustorium or ingested by the insect or nematode.

Said phytopathogens are preferably selected from insects of the lepidopteran or coleopteran type often herbivorous when in the larval state, nematodes and fungi. The herbivorous insect in the adult or larval stage can be *Spodoptera littoralis*, *Heliotis virescens.* Said nematode can be selected from *Meloidogyne incognita, Meloidogyne hapla, Globodera rostochiensis.* Phytopathogen fungi can be selected from oidium, rust, septoriae, venturia, alternaria. These fungi can be diffused in the phylloplane, or in the hypogean parts of the plant, and settle in the surface portions, as also in the more internal tissues.

In the practice of the invention, one or more dsRNA can be constitutively expressed in the tissues of the whole plant, or in certain portions, such as the phylloplane, or in the root system, using specific promoters (tissue-specific promoters). The expression in the hypogean parts of the plant can be aimed at controlling, for example, terrestrial phytopathogens, whereas that in the root system used for the control of nematodes or other harmful insects. Consequently, according to preferred embodiments of the invention, when the dsRNA expression is tissue-specific, said tissue is selected from the groups which consists of phylloplane, trunk, root system, buds, flowers, fruit.

The method described herein envisages the selection of one or more GPCR receptors as RNA interference target, said receptor must be vital for the phytopathogen against which the plant is to be made resistant. In relation to the insect, nematode or fungus various types of GPCR receptor can be used as target. Said GPCR target receptor is preferably selected from the group which consists of allatostatin C or A, octopamin/tyramine, PBAN peptide, diuretic hormone, adipokinetic hormone, neuropeptides, dopamine, serotonin and rhodopsin-like receptors. According to a preferred embodiment, in which, when said herbivorous insect is *Spodoptera littoralis,* the nucleotide sequence of the cDNA encoding a GPCR receptor or a portion thereof is selected from the group which consists of AlstCR (SEQ ID NO:2), DHR (SEQ ID NO:3) or Oct/TyrR (SEQ ID NO:6). With respect to other lepidopteran species, such as for example, *Heliotis virescens,* the authors have found that GPCR receptor homologous to that of allatostatin C of *Spodoptera* could represent the election target for this type of herbivorous insect.

With respect to the use of the method according to the invention for obtaining transgenic plants resistant to nematode parasites, in particular of the species *Meloidogyne incognita,* the authors of the present invention have found two possible GPCR target silencing receptors by means of RNAi. The nucleotide sequence of the cDNA encoding the above GPCR receptors is selected from the group which consists of serotonin-like receptors (homologue of the serotonin receptors of C. elegans, SEQ ID NO:9) and neuropeptide receptor (homologue of the receptor of neuropeptides of *C. elegans* Q2TGX5_MELIC, SEQ ID NO:11).

According to a preferred embodiment of the method of the present invention, one or more dsRNA can be expressed in the tissues of the transgenic plant obtained. This way, it is possible to obtain lines expressing two or three different types of dsRNA and the same plants can therefore be resistant to the attack of both phytophages (which prevalently feed on leaves) and parasites of the roots. Although preserving the specificity of resistance to single species of harmful organisms, plants can be produced, which are resistant to various species, even very different from each other, i.e. belonging to phylogenetically completely distant groups (Phyla). Consequently, phases a) to c) of the method described herein can be repeated in parallel using in each phase, a nucleotide cDNA sequence encoding a GPCR receptor vital for different phytopathogens (i.e. nematode or insect, different species of nematodes, insects or fungi) to obtain one or more recombinant plasmids and d) co-transforming the same plant or dl) transforming different plants and crossing them. Thanks to crossbreeds between transformed plants, plants which express specific dsRNAs both against insect GPCRs (i.e. *Spodoptera, Heliotis*), and against nematode GPCRs (*M. incognita*), can be created as well.

The present invention also envisages the use of the RNA interference technology for protecting plants of agronomical interest (such as, for example, cereals, solanaceous plants, vines, vegetables), ornamental plants (such as roses, etc.), environmental plants (such as conifers, linden trees, poplars, etc.). According to a preferred embodiment of the invention a solanaceous plant is used, preferably tobacco, more specifically *Nicotiana tabacum.*

A further object of the present invention relates to a transgenic tobacco plant resistant to one or more phytopathogens like Spodoptera littoralis and/or Meloidogyne incognita which can be obtained according to the method as defined above. Plants genetically modified for inhibiting GPCR receptors of phytopathogens, according to the procedures envisaged by the present invention, can be used for the production of food destined for human beings or for zootechnics, but also for the production of biomasses for industrial purposes (paper, fibres, pharmacological substances, biomasses for energy production or basic chemical substances, etc.). According to a preferred embodiment of the present invention, said transgenic plant is a tobacco plant resistant to one of its most harmful parasites, the insect *Spodoptera littoralis*. In particular, the transgenic tobacco plant (i.e. *Nicotiana tabacum*) expresses a dsRNA capable of inhibiting a GPCR receptor of *S. littoralis* (selected from AlstCR (SEQ ID NO:2), DHR (SEQ ID NO:3) or Oct/TyrR (SEQ ID NO:6), through the ingestion of plant tissue expressing said dsRNA by the same insect in the larval stage. In a particularly preferred embodiment, the present invention relates to a transgenic plant resistant to *Spodoptera littoralis* characterized in that it is a tobacco plant and expresses dsRNAs in which the antisense strand is complementary to the mRNA encoding the receptor AlstCR (SEQ ID NO:2) or DHR (SEQ ID NO:3). According to another preferred embodiment the invention relates to a transgenic plant resistant to *Meloidogyne incognita* characterized in that it is a tobacco plant and expresses a dsRNA wherein the antisense strand is complementary to the mRNA encoding for SEQ ID NO:11.

An object of the present invention therefore relates to a method for the prophylactic treatment of plants to make them resistant to the attack of one or more insects, nematodes and phytopathogen fungi comprising phases a)-e) of the method defined above. In order to ensure their best development, the plants thus obtained can, if necessary, be subjected to a treatment phase with fertilizers, growth regulators or biostimulants, or with fungicidal products, herbicides, insecticides and nematocides, synthetic or natural. These applications could allow a lesser risk of selecting parasites resistant with respect to the biocide activity obtained with the technology object of this patent, and it is possible to have a synergic action between the application of a pesticide and the biocide activity obtained with the technology object of the present invention.

The present invention will now be described for illustrative but non-limiting purposes, according to its preferred embodiments with particular reference to the figures of the enclosed drawings, in which:
- Figure 1 shows the sequences of the cDNAs encoding the GPCR receptors: AlstAR, AlstCR, DHR, AKHR, LGR1, OctR, OR83b, PBANR cloned from *S. littoralis;*
- Figure 2 shows the sequences of the cDNAs encoding the GPCR receptors: MiSerR1-like, MiDopR1-like and MiNpR1-like cloned from *Meloidogyne incognita;*
- Figure 3 shows the RT-PCR analysis of transgenic plants AlstCR (lanes 1 to 7); WT1 and WT2: non-transformed tobacco plants; M: known molecular weight marker; C+: plasmid pENTR+AlstCR;
- Figure 4 shows the histograms relating to the mortality rate after 4 weeks of *Spodoptera littoralis* larvae fed on transgenic tobacco plants expressing dsRNA specific for AlstCR;
- Figure 5 shows the trend of the mortality rate of *Spodoptera littoralis* larvae fed on transgenic tobacco plants expressing dsRNA specific for AlstCR and DHR;
- Figure 6 shows the histograms relating to the percentage of infected tobacco roots by *M. incognita* of wild-type plants and transgenic plants expressing MiNpR1-like/dsRNA.

For illustrative but non-limiting purposes of the present invention, we describe two examples of transformations of a solanaceous plant, namely tobacco, to protect said plant from two of its most harmful parasites, the insect *Spodoptera* littoralis and the nematode *Meloidogyne incognita.*

### EXAMPLE 1: Transformation of tobacco plants, in order to protect them from the insect Spodoptera littoralis

### MATERIALS AND METHODS

### Selection of GPCR targets

The receptors of *S. littoralis* selected as targets of the present embodiment of the invention are:
1. AlstAR (putative receptor of allatostatin A);
2. AlstCR (putative receptor of allatostatin C):
   these two GPCRs are of fundamental importance as they bind the allatostatins, neuropeptides whose main function is to inhibit the synthesis of the juvenile hormone on the part of the "corpora allata". In addition to being involved in the metamorphosis activation process (Weaver et al., 1994), allatostatins are also important for regulating contractions of the intestinal smooth muscles and in the development and functionality of the ovaries (Aguilar et al., 2003; Meyering-Vos et al., 2006);
3. DHR (receptor of the diuretic hormone DH): important for diuresis regulation (Johnson et al., 2004);
4. PBAN-R (receptor of the activation neuropeptide of the biosynthesis of pheromones, PBAN): it binds a small peptide which promotes the synthesis and release of sexual pheromones (Rafaeli *et al.,* 2007);
5. AKHR (putative receptor of the adipokinetic hormone AKH): important for the metabolism of carbohydrates and lipids and also for flight performances (Staubli *et al,* 2002);
6. LGR1 (Leucine-Rich Repeat-Containing G Protein-Coupled Receptor): belongs to the group of G protein-coupled receptors containing Leucine-rich repetitions (LGR) and exerts a fundamental role in reproduction (Nishi *et al.,* 2000);
7. Oct/TyrR,putative receptor of octopamine and tyramine: it is capable of interacting with neurotransmitters such as octopamine and tyramine and is therefore important in neuromodulation processes in sensorial and secretory organs (Farooqui, 2007);
8. OR83b (receptor of the family of olfactory receptors): unlike other members of the family of olfactory receptors (OR), which are only expressed in small sub-populations of sensorial olfactory neurons, OR83b is expressed in almost all antenna neurons (Neuhaus *et al.,* 2005). Rather than having a direct role in the olfactory function, it interacts with the conventional OR members and is essential for their localization from the cellular bodies to the sensory cilia where interaction with the odorant molecules takes place. For the general role it exerts in the olfactory system, OR83b is certainly of fundamental importance for the perception of odors and therefore for the nutrition of insects.

In order to perform RNAi experiments, all the sequences encoding GPCRs were cloned from *Spodoptera* with the exception of the gene encoding PBAN-R already present in the data bank (AF401480).

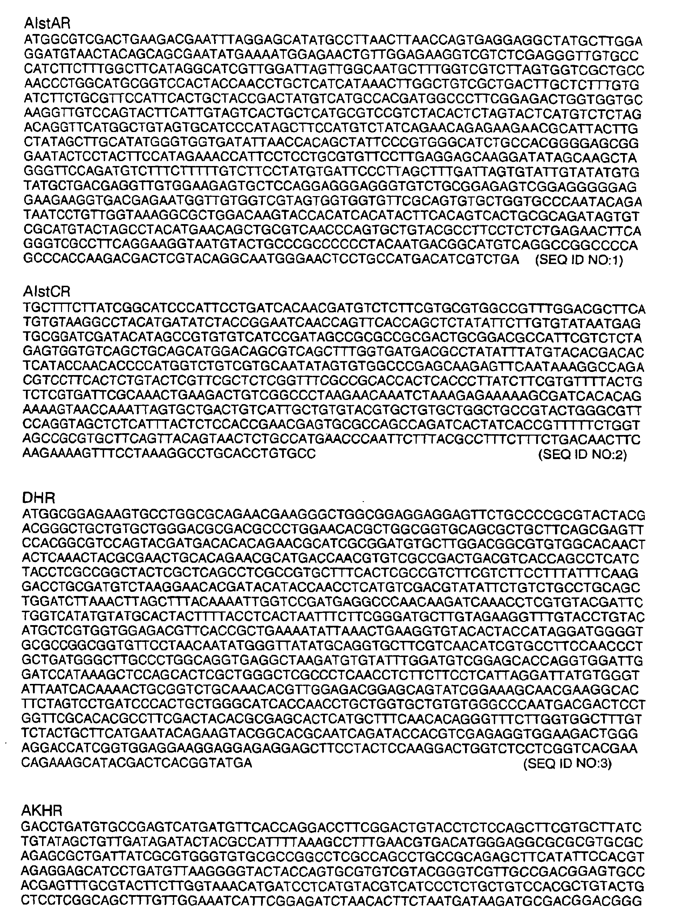

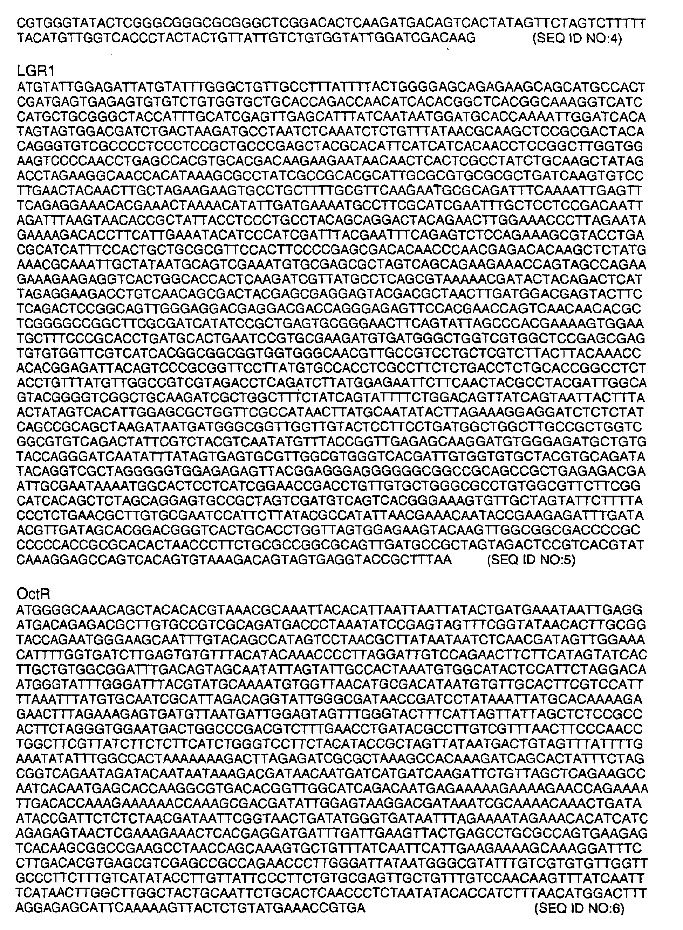

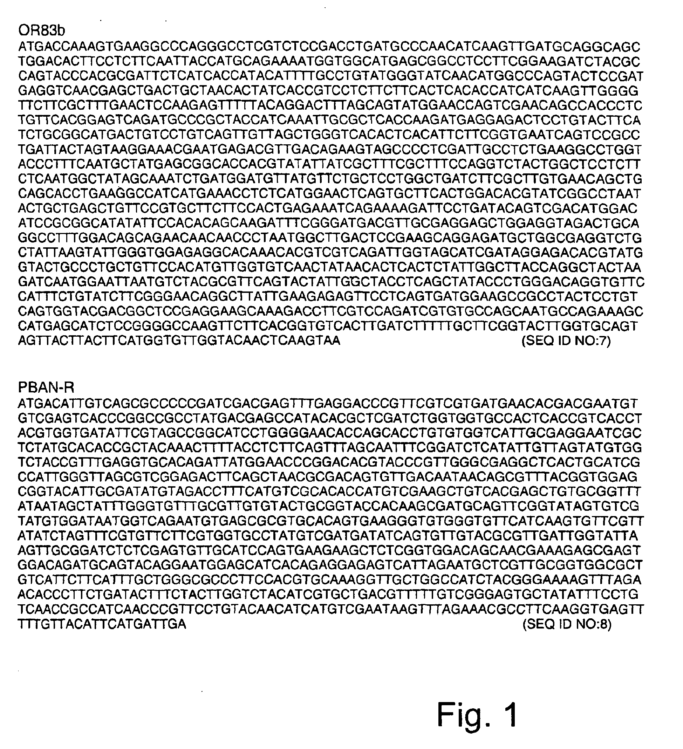

The sequences encoding the receptors AlstAR, DHR, LGR1, OR83b, Oct/TyrR, and part of the sequence encoding the receptor AlstCR and the receptor AKHR, were isolated using the cDNA back-transcribed by the total RNA extracted from *S. littoralis,* using degenerated primers deriving from the comparison of conserved regions among these genes in various insect species. For PBAN-R, on the other hand, specific primers for isolating the encoding sequence starting from V-phase larvae, were used. For the other receptors, on the basis of expression data available in literature, the sequences were isolated using different tissues and growth phases of the insect. In particular, the gene encoding the receptors LGR was amplified from eggs, whereas DHR from V-phase larvae; the gene encoding the receptors AlstAR, AlstCR, AKHR, OR83b, Oct/TyrR were amplified by RNA extracted from the head of individual adults. The PCR products were sequenced and used for designing specific primers for the 5' and 3' RACE-PCR experiments. The isolated sequences were cloned in the plasmid pCR^{®} 2.1-TOPO^{®} (*Invitogen*) and entirely sequenced (Figure 1).

### Isolation and cloning of the selected receptors

### Cloning of the receptor AlstAR

In order to isolate the cDNA encoding the receptor of Allatostatin A of *Spodoptera littoralis,* degenerated primers were designed on conserved regions determined by the alignment among the amino acid sequences of homologous receptors of *Bombyx mori, Drosophila melanogaster e Periplaneta americana.* The sequences of the primers are indicated below (the nucleotides in brackets indicate degenerated positions; the primer i.e. is a mixture of oligonucleotides, each containing a different nucleotide in that specific position):
1Fw: atg (ac) g (acgt) (at) (gc) (acgt) ac (agct) ac (agct) aa (tc) (tc) t (agct) (tc) t (agct) at (tc) a (ag) (tc) (SEQ ID NO:12)
2Fw: gt (agct) cc (agct) tt (tc) ac (agct) gc (agct) ac (agct) ga (tc) ta (tc)gt(agct)atg (SEQ ID NO:13)
1Rv: gt(agct)ac(agct)(ac)g(agct)atggt(agct)gt(agct)gt(agct) gt(agct)gt(agct) (SEQ ID NO:14)
2Rv: tgg (at) s (agct) tg (tc) gt (agct) aa (tc) cc (atcg) gt (atgc) (ac) t(tc)ta(tc)gc(agct) (SEQ ID NO:15).

The total RNA was extracted from heads of individual adults of *Spodoptera littoralis* using a kit commercialized by Promega. The samples of RNA were subjected to a treatment with rDNasi1 (Ambion) to eliminate the contaminating genomic DNA. 2 µl of RNA were charged onto 1% agarose gel in presence of denaturing loading dye and quantified using a specific marker for RNA (Fermentas) as reference. Gene tools software (Perkin Elmer) was used for the quantification. 1 µg of total RNA was transcribed into cDNA using the RevertAid M-MuLV Reverse Transcriptase enzyme (Fermentas) and 500 ng of oligodT (Promega).

A fragment of 507 bp was obtained from the amplification of the cDNA through 30 PCR cycles (10 s at 98°C, 30 s at 50°C, 30 s at 72°C) followed by 10 extension minutes at 72°C. The reaction was performed in a volume of 50 µl containing each primer at a concentration of 2.5 µM, 0.2 mM of deoxynucleotides (Fermentas) and 1 unit of Phusion High-Fidelity DNA Polymerase (Finnzymes) in the corresponding buffer.

The fragment obtained was cloned in the cloning vector pCR2.1 (Invitrogen) and subsequently sequenced (Ceinge Biotecnologie Avanzate scarl) using the oligonucleotides M13Fw and M13Rv, specific for the vector.

On the basis of the sequence, the following groups of oligonucleotides were designed for the 3' RACE (Group 1; sense) and 5' RACE (Group 2; antisense) respectively:
Group 1:
   1s GCATCCCATAGCTTCCATGT (SEQ ID NO:16)
   2s GGTGATATTAACCACAGCTATTCCCGTGGGC (SEQ ID NO:17)
   3s GTATGCTGACGAGGTTGTGGAAGAGTGCTCC (SEQ ID NO:18)
   4s AAGGTGACGAGAATGGTTGTG (SEQ ID NO:19)
   5s GCGCAGATAGTGTCGCATGTA (SEQ ID NO:20)
Group 2:
   1as AGACGACTCTTCCACAACCTCGTCAGCATAC (SEQ ID NO:21)
   2as GTTAATATCACCACCCATAT (SEQ ID NO:22)
   3as GCCCACGGGAATAGCTGTGGTTAATATCACC (SEQ ID NO:23).

The 5' and 3' ends of the gene were obtained by means of PCR-RACE using the 5'/3' RACEKIT in which the anchor primer and oligo(dT) anchor primer were substituted with oligo SL1 and oligo(dT)SL1 whose sequences are indicated hereunder:
SL1 GGTTTAATTACCCAACTTTGAG (SEQ ID NO:24)
dTSL1 GGTTTAATTACCCAACTTTGAGTTTTTTTTTTTTTTTT (ACG) (SEQ ID NO:25).

1 µl of cDNA, synthesized using 1.87 µM of the primer dTSL1, was amplified with the primers 1s and SL1 to obtain the 3' end of the gene. The amplification conditions were: 40 cycles comprising 10 s at 98°C, 30 s at 46°C, 30 s at 72°C followed by 10 extension minutes at 72°C. Subsequent amplifications were effected using the primer SL1 in a combination with each of the primers of Group 1 using the above conditions as amplification conditions.

A fragment of 650 bp was obtained, cloned in the pCR2.1 and sequenced as described above.

In order to amplify the 5' end, the protocol indicated by the supplier of the Kit was followed, using the primer 1as for the synthesis of the cDNA and the primers 2as-3as and SL1 for the amplifications. A fragment of about 500 bp was obtained, cloned and sequenced.

In order to obtain the cDNA encoding the whole gene, 1 µl of cDNA used for the reactions with the degenerated primers was amplified, with the following primers:
5'-ATGGCGTCGACTGAAGAC-3' (SEQ ID NO:26)
3'-TCAGACGATGTCATGGCA-5' (SEQ ID NO:27).

The amplification conditions used were the following: 40 cycles comprising 10 s at 98°C, 30 s at 60°C, and 1 minute at 72°C followed by 10 extension minutes at 72°C. A fragment of about 1080 bases was obtained, cloned in the vector pCR2.1 and sequenced.

### Cloning of the receptor OR83b

In order to isolate the cDNA encoding the receptor OR83b of *Spodoptera littoralis,* a specific reverse primer and a degenerated forward primer were designed. The degeneration of the forward primer interested only one position since the alignment between the respective nucleotide sequences deriving from *Spodoptera exigua, Heliothis virescens, Helicoverpa* zea and *Mamestra brassicae* revealed a very high preservation degree among each other. The sequences of the primers are indicated hereunder:
Fw ATGACCAA(AG)GTGAAGGCCCAG (SEQ ID NO:28)
Rv GTGTTGGTACAACTCAAGTAA (SEQ ID NO:29).

The cDNA prepared as described in the previous paragraph was used, and of this 1 µl was amplified with 250nM of each of the two primers in 30 cycles comprising 10 s at 98°C, 30 s at 50°C, and 1.5 minutes of extension. A fragment of about 1,500 bp was obtained, which was cloned in the vector pCR2.1 and sequenced as described above.

### Cloning of the receptor DHR

In order to isolate the cDNA of the receptor DHR from *Spodoptera littoralis,* degenerated primers were designed on the basis of the alignment among the corresponding protein sequences of *Manduca sexta, Drosophila melanogaster, Nilaparvata lugens* e *Acheta domesticus.* The sequences of the primers used are the following:
1Fw: TT (TC) (TC) T (ATCG) TA (TC) TT (TC) AA (AG) GA (ATCG) (TC) T (ATG) (AC) G (ATCG) TG (TC) (SEQ ID NO:30)
2Rv: A (AG) (TC) TT (ATCG) GT (ATCG) AT (ATCG) A (AG) (ATCG) ACCCACAT (ATCG)AT (SEQ ID NO:31).

RNA was extracted from V-phase larvae with the method already described. For the quantification of the RNA and synthesis of the cDNA, the protocols described for the cloning of AlstAR were used.

A fragment of 550 bp, was obtained through 45 amplification cycles, comprising 10 s at 98°C, 30 s at 48°C, and 1 minute at 72°C followed by 10 extension minutes at 72°C, which was subsequently cloned in the vector pCR2.1 and sequenced.

On the basis of the sequence, specific primers were designed and used for the subsequent 3' and 5' RACE reactions following the method already described. The sequences of the primers are the following:
Group 1:
   1s AACCTCATGTCGACGTATATTCTGTCT (SEQ ID NO:32)
   2s ATGCTTGTAGAAGGTTTGTACCTGTAC (SEQ ID NO:33)
   3s TGGGTTATATGCAGGTGCTTCGTCAAC (SEQ ID NO:34)
Group 2:
   1as GGATGGGGTGCGCCGGCGGTGTTCCTA (SEQ ID NO:35)
   2as CATACATATGACCAGAATCGTACACGA (SEQ ID NO:36)
   3as GGCGAGGTAGATGAGGCTGGTGACGTC (SEQ ID NO:37).

For 3', a fragment of about 650 bp was isolated through 40 amplification cycles, comprising 10 s at 98°C, 30 s at 46°C, 1 minute at 72°C and followed by 10 extension minutes at 72°C. For 5', a fragment of about 500 was obtained through 40 amplification cycles according to the procedure described above.

The following primers were used for the isolation of the entire cDNA:
5' -ATGGCGGAGAAGTGCCTGGCG-3' (SEQ ID NO:38)
3'-TCATACCGTGAGTCGTATGCT-5' (SEQ ID NO:39).

A fragment of 1190 bp was amplified by 1 µl of cDNA, synthesized by RNA extracted from V-instar larvae, with the Fw and Rv primers through 45 amplification cycles, comprising 10 s at 98°C, 30 s at 55°C, 30 s at 72°C and followed by 10 extension minutes.

### Cloning of the receptor LGR1

In order to isolate the cDNA of the receptor LGR1 from *Spodoptera littoralis,* degenerated primers were designed on the basis of the alignment among the corresponding protein sequences of *Bombyx mori, Drosophila melanogaster* e *Aedes aegypti.* The sequences of the primers used were the following:
1Fw: GC (ATGC) TA (TC) (TC) T (AGCT) AC (AGCT) CA (TC) (GC) (AGCT)
(AGCT)TT(TC)CA(TC)TG(TC)TG(TC) (SEQ ID NO:40)
1Rv: TC (AGCT) A (TC) (AGCT) GG (AGCT) A (AG) (AG) CA (AGCT) AT (AGCT) (GC) (AT) (AGCT) GT (SEQ ID NO:41).

RNA was extracted from the eggs of *Spodoptera littoralis,* following the method already described. For the quantification of the RNA and synthesis of the cDNA, the protocols described for the cloning of AlstAR were used.

A fragment of about 1,250 bp, was obtained through 45 amplification cycles, comprising 10 s at 98°C, 30 s at 48°C, 1.5 minutes at 72°C and followed by 10 extension minutes at 72°C, which was subsequently cloned in the vector pCR2.1 and sequenced.

From an analysis of the sequence, the following primers were designed, subsequently used for the 3' and 5' RACE reactions with the methods previously described:
Group 1:
   1s TGATGAAAATGCCTTCGC (SEQ ID NO:42)
   2s CACGAACCAGTCAACAACAC (SEQ ID NO:43)
   3s GAAAGCGTACCTGACGCATCATTTCCA (SEQ ID NO:44)
   4s GTTATCAGTAATTACTTTAACTATAGT (SEQ ID NO:45)
Group 2:
   1as ACTGCCGGAGTCTGAGAAGTA (SEQ ID NO:46)
   2as GTACTCCTCGCTCGTAGTCG (SEQ ID NO:47)
   3as CATTTCGACTGCATTATAGC (SEQ ID NO:48)
   4as GCCTTCTAGGTCTATAGCTTG (SEQ ID NO:49).

A fragment of about 500 bp and a fragment of 860 bp were isolated, cloned in the vector pCR2.1 and sequenced at 3' and 5' respectively, through 35 amplification cycles on the cDNA synthesized starting from RNA extracted from the eggs of *Spodoptera littoralis,* according to the procedures described in the previous paragraphs.

In order to isolate the cDNA corresponding to the entire sequence encoding the receptor LGR1 from *Spodoptera littoralis,* the following primers were used, corresponding to the ends 5' and 3' respectively:
5'-ATGTATTGGAGATTATGTATTTGGGCT-3' (SEQ ID NO:50)
3'-TTAAAGCGGTACCTCACTACTGTCTTT-5' (SEQ ID NO:51).

A fragment of 2,250 bp was isolated, cloned and sequenced through standard amplification procedures.

### Cloning of the receptor Oct/TyrR

For the isolation of the cDNA encoding the receptor Oct/TyrR from *Spodoptera littoralis,* degenerated primers were designed on the basis of the alignment among the nucleotide sequences of the corresponding genes of *Bombyx mori, Heliothis virescens* and *Mamestra brassicae.*

The sequences of the primers used are the following:
1Fw CCAGAATGGGA(AG)GC(AT)AT(TC)TGCAC (SEQ ID NO:52)
2Rv AC (AG) CCCATTAT (TG) AT (AG) CC (TC) AG (AG) G (SEQ ID NO:53).

The primers were used for amplifying a fragment of 1,090 bp from cDNA synthesized on RNA extracted from heads of individual adults, prepared according to the methods described above. Once the fragment had been cloned in the vector pCR2.1 and sequenced, the following primers, used for the 3' and 5' RACE reactions, were designed:
Group 1:
   1s CCAGAAAATTGACACCAA (SEQ ID NO:54)
   2s GAGAGTAACTCGAAAGAAAC (SEQ ID NO:55)
   3s TGCTGTTTATCAATTCATTGAAGA (SEQ ID NO:56)
Group 2:
   1as AGTTCTTTTTTTAGTGGCCAAA (SEQ ID NO:57)
   2as GACAAGGCGTATCAGGTTC (SEQ ID NO:58)
   3as ACCCTAGAAGTGGCGGAGAGCTAATA (SEQ ID NO:59).

A fragment of about 380 bp and a fragment of 590 bp were isolated, cloned in the vector pCR2.1 and sequenced at 3' and 5' respectively, through 40 amplification cycles on the cDNA synthesized starting from RNA extracted from heads of individual adults of *Spodoptera littoralis,* according to the procedures described in the previous paragraphs.

In order to isolate the cDNA corresponding to the entire sequence encoding the receptor Oct/TyrR, the following primers were used:
5'-ATGGGGCAAACAGCTACACAC-3' (SEQ ID NO:60)
3' CTCTGTATGAAACCGTGA-5' (SEQ ID NO:61).

A fragment of 1,434 bp, was isolated, cloned and sequenced through 40 amplification cycles, comprising 10 s at 98°C, 30 s at 55°C and 1 minute at 72°C.

### Cloning of the receptor PBANR

The sequence of the receptor PBANR was already available in data bank, consequently primers corresponding to 5' and 3' of the encoding region were designed, whose sequences are indicated hereunder:
5'-ATGACATTGTCAGCGCCCCCGATC-3' (SEQ ID NO:62)
3'-TCAATCATGAATGTAACA-5' (SEQ ID NO:63).

The primers were used for amplifying 1 µl of cDNA synthesized starting from RNA extracted from V-instar larvae, according to the procedures described. The amplification was effected for 40 cycles of 10 s at 98°C, 30 s at 55°C, 40 s at 72°C, followed by 10 extension minutes at 72°C. A fragment of 1,053 bp was obtained, which was subsequently cloned in the vector pCR2.1 and sequenced according to the procedures described.

### Cloning of part of the sequence encoding the receptor AlstCR

For the isolation of the receptor AlstCR of *Spodoptera littoralis,* the following degenerated primers were designed on the basis of the homology among the respective protein sequences of *Apis mellifera, Drosophila melanogaster* and *Anopheles gambiae:*
1Fw: GA (AG) TG (TC) TT (TC) (TC) T (AGCT) AT (ATC) GG (ATGC) (SEQ ID NO:64)
1Rv : (ATGC) GC (AG) CA (ATGC) GT (AG) CA (ATGC) GC (TC) TT (SEQ ID NO:65).

The primers 1Fw and 1Rv were used for amplifying cDNA transcribed from RNA extracted from heads of individual adults, through 40 amplification cycles at 45°C. A fragment of 726 bp was isolated, subsequently cloned in the vector pCR2.1 and sequenced.

Cloning of part of the sequence encoding the receptor AKHR For the isolation of the receptor AKHR of *Spodoptera littoralis*, the following degenerated primers were designed on the basis of the homology between the nucleotide sequences of the corresponding receptors of *Bombyx mori* and *Periplaneta Americana*:
1Fw: GACCTGATGTGC(AC)G(AC)(AG)TCATG (SEQ ID NO:66)
1Rv: GTC(AG)ATCCA(AG)TACCACAG(AG)CA (SEQ ID NO:67).

The primers 1Fw and 1Rv were used for amplifying cDNA transcribed from RNA extracted from heads of individual adults, through 40 amplification cycles at 45°C. A fragment of 534 bp was isolated, subsequently cloned in the vector pCR2.1 and sequenced.

### Cloning of the receptors in the plasmids pENTER and PH7GW1WG2

The genes encoding the receptors AlstAR, AlstCR, DHR, AKH, OR83b and Oct/TyrR were isolated from the plasmids pCR^{®} 2.1-TOPO^{®} by digestion or by PCR using a primer forward and a primer reverse homologous to the sequence of the gene to be isolated, carrying the recognition sequence for the restriction enzymes EcoRI (forward primer) and XhoI (reverse primer), compatible with the polylinker of the plasmid pENTR. LGR1 receptor receptor was amplified by the plasmid pCR^{®} 2.1-TOPO with a forward primer carrying the recognition sequence for BamHI enzyme and a reverse primer containing the XhoI site. For cloning PBAN receptor a forward primer containing the EcoRI site was used in combination with a reverse primer carrying the sequence cut by NotI enzyme. The sequences of the primers are indicated hereunder:
AlstAR Fw 5'-GGAATTCCATGGCGTCGACTGAAGAC-3' (SEQ ID NO:68)
AlstAR Rev 5'-CCTCGAGGTCAGACGATGTCATGGCA-3' (SEQ ID NO:69)
AlstCR Fw 5'-GGAATTCCCTGCGTTCCATTCACTGCTA-3' (SEQ ID NO:70)
AlstCR Rev 5'-CCTCGAGGAAGAATTGGGTTCATGGCAG-3' (SEQ ID NO:71)
DHR Fw 5'-GGAATTCCGATGGCGGAGAAGTGCCTGGC-3' (SEQ ID NO:72)
DHR Rev 5'-CCTCGAGGGTCATACCGTGAGTCGTATGC-3' (SEQ ID NO:73)
AKH Fw 5'-GGAATTCCGGACCTGATGTGCCGAGTCATG-3' (SEQ ID NO:74)
AKH Rev 5'-CCTCGAGGGCTTGTCGATCCAATACCAC-3' (SEQ ID NO:75)
LGR1 5'-CGGGATCCCGGATGTATTGGAGATTATGTATTTGGGC-3' (SEQ ID NO:76)
LGR1 Rev 5'-CCTCGAGGGTTAAAGCGGTACCTCACTAC-3' (SEQ ID NO:77)
OR83b Fw 5'-GGAATTCCGATGACCAAAGTGAAGGCCCAGG-3' (SEQ ID NO:78)
OR83b Rev 5'-CCTCGAGGGTTACTTGAGTTGTACCAACACC-3' (SEQ ID NO:79)
Oct/TyrR Fw 5'-GGAATTCCGGGGCAAACAGCTACACACG-3' (SEQ ID NO:80)
Oct/TyrR Rev 5'-CCTCGAGGGTCACGGTTTCATACAGAGTAAC-3' (SEQ ID NO:81)
PBAN-R Fw 5'-GGAATTCCGATGACATTGTCAGCGCCCCCG-3' (SEQ ID NO:82)
PBAN-R Rev 5'- TAAAGCGGCCGCTCAATCATGAATGTAACAAAAA-3' (SEQ ID NO:83).

The amplification reaction was performed in a final volume of 50 µl using 50 ng of plasmid DNA, 200 µM of dNTPs, 0.25 µM primer forward, 0.25 µM primer reverse, 5x Phusion HF buffer, 1U of Phusion High-Fidelity DNA Polymerase (Finnzymes). The amplification program consists in a denaturing cycle at 98°C for 30 s, 35 cycles at 90°C for 10 s, 52°C for 30 s, 72°C for 1 min and one final extension cycle at 72°C for 10 min. About 5 µl of amplified product were separated on agarose gel at 1% to verify its quality and molecular weight. After purification from the reagents used for the amplification (GenElute^{™} PCR Clean-Up Kit, SIGMA) the remaining PCR reactions were subjected to digestion with 20U of appropriate restriction enzymes to produce sticky ends for the subsequent cloning in the vector pENTR, also digested with the same restriction endonucleases. The ligation reaction between the genes of the receptors (200 ng) and the vector pENTR (300 ng) were performed in a volume of 20 µl at 16°C, using 20u of T4 DNA ligase (Biolabs). 10 µl of ligase reaction were used for transforming competent cells of *E*. *coli* DH5α plated on LB with the antibiotic kanamycin (50 mg/l) to select the cell clones carrying the pENTR plasmids. The colonies obtained were inoculated with liquid LB and kanamycin at 37°C for the whole night.

The plasmid DNA was extracted (Wizard^{®} Plus SV Minipreps DNA Purification System, Promega) and digested with 10u of suitable restriction enzymes.

The pENTR plasmids with the cloned receptors were used for the recombination reaction with the binary vector pH7GW1WG2(I). 150 ng of pENTR plasmid were incubated with 300 ng of pH7/GW1WG2(I) at 25°C in the presence of the enzyme Gateway^{®} LR clonase II Enzyme Mix (*Invitrogen*) for two hours. The reaction was then blocked with Proteinase K at 37°C for 10 min. and used for transforming the DH5α cells. The plasmids were analyzed by digestion with restriction enzymes and further verified by means of sequence analysis. Competent cells of *Agrobacterium tumefaciens* (C58) were transformed with the recombinant plasmids with the freezing/defrosting technique. About 10 µg of plasmid were added to 100 µl of competent cells and incubated in ice for 5 minutes. The cells were put in dry ice/ethanol for 5 minutes and then transferred at 37°C for 10 minutes. 1 ml of LB was added to the cells and they were incubated at 28°C for 3 hours before being plated on LB containing Rifampicin 100 mg/l and Spectinomycin 100 mg/l.

The resistant colonies were analyzed by means of PCR with primers specific for each cloned receptor.

### Transformation of tobacco plants with dsRNA

The Gateway technique was used for the expression of the dsRNA in tobacco plants: the sequences encoding the receptors were isolated from the pCR^{®} 2.1-TOPO^{®} plasmids in which they were cloned and sequenced. The PCR reactions were effected using oligonucleotides complementary to the gene sequence and carrying the sites recognized by the restriction enzymes for the cloning of the plasmid pENTR (Invitrogen). This plasmid is an Entry Vector which, in addition to the cloning of the DNA sequences, allows them to be transferred into the expression vector (Destination Vector) thanks to the presence of two specific recombination sites (attL1 e attL2) which flank the cloned PCR product. The recombinant clones were used for the recombination reaction with the binary vector pH7GWIWG(I) (Plant System Biology) for the plant expression. This plasmid is characterized by the presence of the promoter 35S for the constitutive expression of the gene cloned downstream of the sites attR1 and attR2, which serve for recombination with the Entry Vector and transfer of the 5'-3' strand of the cloned gene upstream of an intronic sequence and downstream of another identical intronic sequence. The presence of the constitutive promoter ensures the expression of the gene whereas the intronic sequence allows the formation of the dsRNA. The recombination reaction between the sites attL1 and attL2 of pENTR and the sites attR1 and attR2 of pH7GWIWG(I) is mediated by the enzyme Gateway^{®} LR Clonase^{™} (Invitrogen). The recombinant plasmids were characterized by means of digestion with restriction endonucleases and further verified by sequencing. These plasmids were transferred into competent cells of Agrobacterium tumefaciens (strain C58) for the transformation of tobacco plants. For each construct, about 50 leaf disks were co-cultivated and the same quantity co-cultivated with agrobacterium transformed with the empty plasmid pH7GWIWG(I) as control for the subsequent biological tests.

After transformation of *Nicotiana tabacum* plants with *Agrobacterium tumefaciens* carrying the plasmid pH7GW1WG2 expressing the single cloned receptors, the leaf explants were co-cultivated for 3 days with 100 µl of bacterial culture grown at 28°C until reaching a DO₆₀₀=0.8 in the presence of 10 ml of liquid medium A10 (3.6 g/l B5, 250 mg/l NH₄NO₃, 500 mg/l MES, 2% Glucose, 1 µg/ml benzylaminopurine, 0.1 µg/ml naphthalene-acetic acid, pH 5.7). The Agrobacterium was removed by rinsing the pieces of leaf with fresh A10 medium containing cefotaxime 500 µg/ml. The leaf disks were then transferred onto solid A11 medium in the presence of the antibiotic hygromycin (30 mg/l). The explants were transferred every week onto fresh medium until the formation of calluses which were put on A12 (All with a concentration of cefotaxime equal to 200 µg/ml). The shoots were transferred onto solid MS30 (Murashige and Skoog 4.4 g/l, Saccharose 30 g/l pH 5.7) and hygromycin.

### Characterization of the transgenic plants

The genomic DNA was extracted from 0.5 g of leaves of tobacco plants grown on selective medium (GenElute^{™} Plant Genomic DNA miniprep Kit, Sigma) and used as a mould for verifying the presence of exogenous DNA by means of PCR. The plants positive to PCR were further characterized for the presence of dsRNA by extracting the RNA from the leaves of the transgenic plants (GenElute^{™} Mammalian Total RNA Miniprep Kit, Sigma) for the reverse-transcription of the cDNA. For this purpose, 2 µg of total RNAs were incubated with 250 ng of Random hexamers (Invitrogen) at 70°C for 5 minutes and then in ice for 5 minutes. A solution containing 1 mM of dNTP, 1X Reaction buffer, 20u of Ribonuclease inhibitor was added to the reaction and the mixture was incubated at 25°C for 5 minutes. 200u of RevertAid^{™} M-MuLV Reverse were added to the samples and the reaction incubated at 25°C for 10 minutes, then at 42°C for 60 minutes and then for 10 minutes at 70°C. 1 µl of cDNA is used for the PCR reactions and for each receptor the primers used for cloning in pENTR.

### Tests with larvae of S. littoralis

For the biological tests on with dsRNA/AlstC-R and dsRNA/DHR transgenic plants, instar-III larvae of *Spodoptera littoralis* were used. Each larva was placed in a container having a diameter of 6 cm in which pieces of leaf of each transgenic line were introduced daily. As control, larvae of the same age were fed with leaves of plants transformed with the empty plasmid, and thus not expressing any dsRNA.

### RESULTS

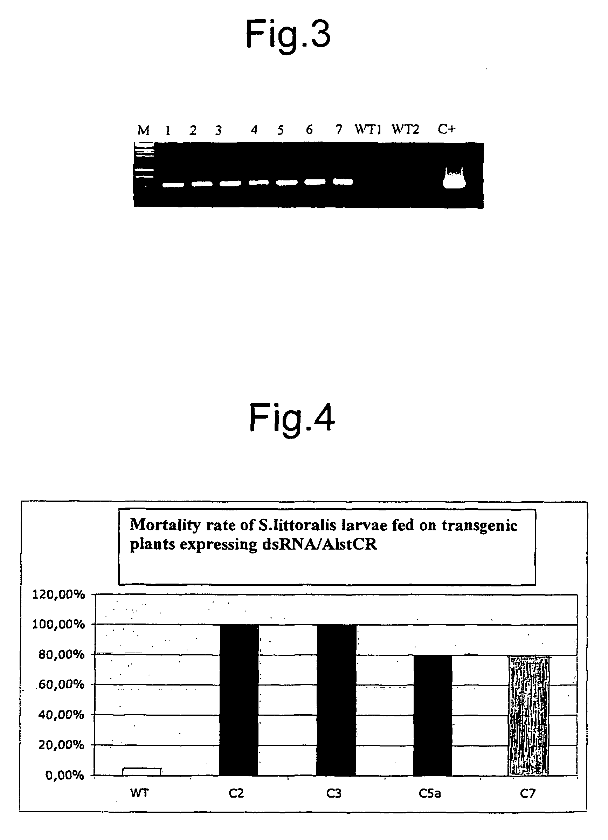

The plants regenerated on a selective medium were characterized for the presence of the heterologous gene by means of PCR on genomic DNA extracted from the leaves and the production of the dsRNA was verified by means of RT-PCR on cDNA reverse-transcribed from total RNA. Figure 3 shows the RT-PCR analysis of the dsRNA on AlstC-R transgenic plants. In all the 7 lines analyzed, the expression level of dsRNA was quite high, while in the control plants (WT1 and WT2) no expression was detected. To make sure the amplification reaction was working correctly, a positive control (C+) of a plasmid containing the AlstCR gene was used as template. Analogous results were obtained for the plants expressing the dsRNA of other receptors (AlstA-R, DHR, PBAN-R, OctR). All the positive transgenic plants were micropropagated by nodal cuttings in order to obtain homogeneous populations of single transformants to be tested with the larvae.

The tests with larvae were carried out using instar-I larvae of *S. littoralis* fed on pieces of leaf of transgenic plants grown in sterile conditions. The larvae were raised together until instar III and then each one was isolated in 10 cm-petri dish where pieces of leaves were added daily for the whole life cycle of the insect until reaching the pupa phase.

In a first preliminary experiment, 20 larvae for each transgenic line, were used in the test. The graph in Figure 4 shows the result obtained from the biotest on plants expressing dsRNA for AlstC-R. The transgenic lines analyzed (C2, C3, C5a and C7) are indicated on the axis of the abscissa whereas the mortality percentage on the ordinates. It is evident that the larvae fed on transgenic plants show a much higher mortality percentage with respect to the larvae fed on the control plants (WT). While the larvae fed on the control showed a mortality rate of around 3%, those fed on the transgenic lines had around 80% mortality (lines AlstC-R 5a and 7) and 100% (lines AlstC-R 2 and 3), after 4 weeks of feeding.

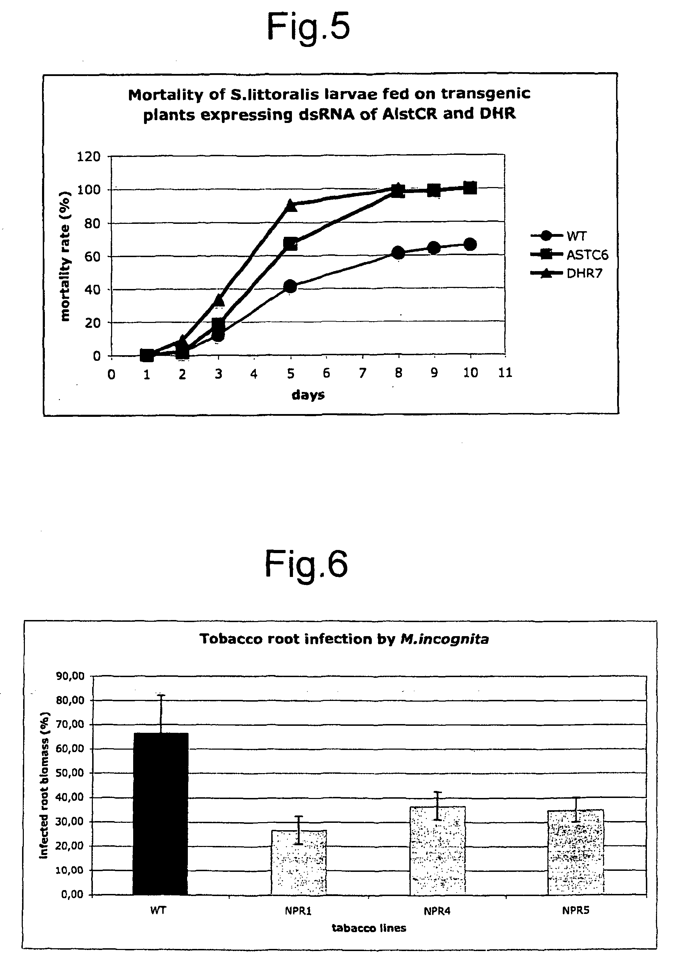

In a second experiment, the mortality rate of larvae fed on two transgenic lines (expressing dsRNA of AlstCR and DHR, respectively) was tested and compared to control larvae fed on wt plants. The mortality rate of the larval population was measure over 10 days, starting from instar I larvae. In the graph showed in Figure 5, the mortality rate of the larvae fed on both the transgenic lines reach 100% after 8 days, while in the control is still 60%. This experiment clearly indicated that the presence of dsRNA, both AlstCR and DHR, in the plants produced a significant reduction of the vitality of the larvae, confirming the validity of the technology proposed.

### EXAMPLE 2: Transformation of tobacco plants, in order to protect them from the nematode Meloidogyne incognita

### MATERIALS AND METHODS

### Selection of the GPCR targets

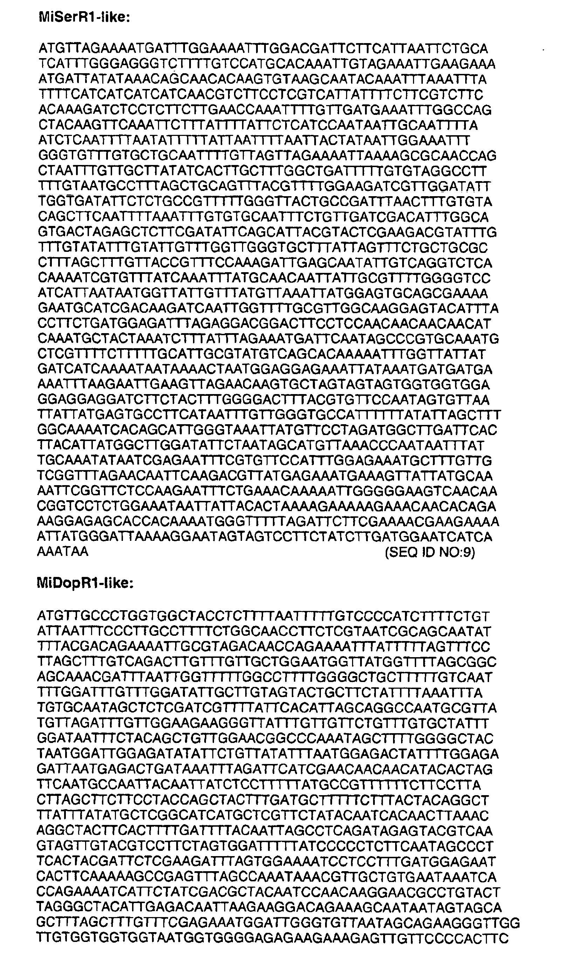

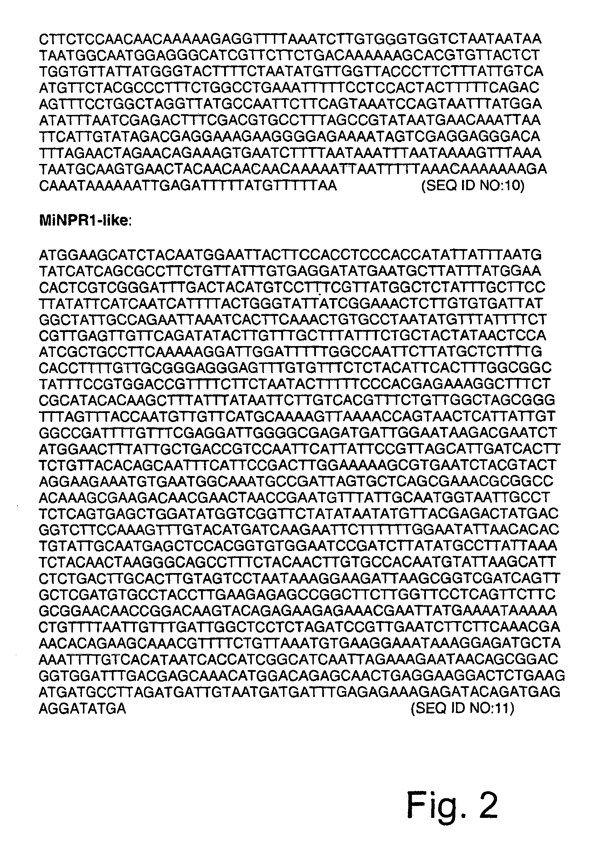

The GPCR receptors of *M. incognita* which were cloned from this organism and selected as targets of the present embodiment of the invention are the following:
1. a homologue of the serotonin receptors of *C*. *elegans,* called MiSerR1-like (Figure 2; SEQ ID NO:9);
2. a homologue of the CeDop-1 dopamine receptor of *C. elegans,* called MiDopR1-like (Figure 2; SEQ ID NO:10);
3. a homologue of the CeNPR1-like neuropeptide receptor of *C. elegans,* called MiNPR1-like (Figure 2; SEQ ID NO:11);
4. a homologue of a putative GPCR of *C. elegans* (T27D1.3) (belonging to the family of Rhodopsin-like GPCRs), called MiRho1-like;
5. a homologue of a putative GPCR of *C. elegans* (T07D4.1) (belonging to the family of Rhodopsin-like GPCRs), called MiRho2-like;
6. a homologue of a putative GPCR of *C. elegans* (B0563.6) (belonging to the family of Rhodopsin-like GPCRs), called MiRho3-like;
7. a homologue of a putative GPCR of *C. elegans* (F02E8.2) (belonging to the family of Rhodopsin-like GPCRs), called MiRho4-like.

In addition to these 6 receptors, another sequence corresponding to a GPCR of interest was identified in a data bank: MiNpR1-like (Q2TGX5_MELIC) which is a homologue of a neuropeptide receptor of the model nematode *C. elegans* (Figure 2, SEQ ID NO:11).

Tobacco plants were transformed with plasmids containing gene sequences (which produce dsRNA in plants) of the receptors MiSerR1-like (SEQ ID NO:9), MiDopR1-like (SEQ ID NO:10) and MiNpR1-like (SEQ ID NO:11). The nucleotide sequences of the receptors which were analyzed are indicated in Figure 2.

### Cloning of the receptors of M. incognita

In order to clone the receptors MiSerR1-like and MiDopR1-like from *M. incognita,* the data bank of the expressed sequences of the nematode *M. incognita* (available at http://www.nematode.net/BLAST/Cluster.BLAST /index.php) was interrogated with various peptide sequences of the homologous receptors of *C. elegans.*

The clones identified were:
- AW 735607 for MiSerR1-like
- AW 571066 for MiDopR1-like.

Starting from the sequences of the identified clones, specific oligonucleotides were designed, and used for amplifying portions of the receptor sequences. The sequences of the whole receptors were then obtained using the 5' and 3' Race method, previously described. With respect to the MiNpr1-like receptor, the cloning was performed using specific primers at 5' and 3' of the gene whose sequence was already present in data banks (Q2TGX5 MELIC). The oligonucleotides used for the amplification are the following:
For MiSerR1-like (SEQ ID NO:9):
   MiserR F1: GATTTGGAAAATTTGGACGAT (SEQ ID NO:84)
   MiserR F2: GGAGGGTCTTTTGTCCATGCA (SEQ ID NO:85)
   MiserR F3: TCTCATCCAATAATTGCAATT (SEQ ID NO:86)
   MiserR R1: ACGTAATGCTGAATATCGAAG (SEQ ID NO:87)
   MiserR R2: CAAATTTAAAATTGAAGCAGT (SEQ ID NO:88)
   MiserR R3: AGCCAAAGCAAGTGATATAAG (SEQ ID NO:89)
For MiDopR1-like (SEQ ID NO:10):
   MidopR F1: ACTCTTGGTGTTATTATGGGC (SEQ ID NO:90)
   MidopR F2: TGGCTAGGTTATGCCAATTCT (SEQ ID NO:91)
   MidopR F3: CGAGACTTTCGACGTGCCTTT (SEQ ID NO:92)
   MidopR R1: AAAGGCACGTCGAAAGTCTCG (SEQ ID NO:93)
   MidopR R2: AGAATTGGCATAACCTAGCCA (SEQ ID NO:94)
   MidopR R3: GCCCATAATAACACCAAGAGT (SEQ ID NO:95).

The following oligonucleotides were used for the amplification of the whole encoding region:
MiserR Forward: ATGTTAGAAAATGATTTGGAAAA (SEQ ID NO:96)
MiserR Reverse: TTATTTTGATGATTCCATCA (SEQ ID NO:97)
MiDopR Forward: ATGTTGCCCTGGTGGCTACCTCT (SEQ ID NO:98)
MiDopR Reverse: TTAAAAACATAAAAATCTCA (SEQ ID NO:99)
MiNprR Forward: ATGGAAGCATCTACAATGGAATT (SEQ ID NO:100)
MiNprR Reverse: TCATATCCTCTCATCTGTAT (SEQ ID NO:101).

The receptors thus amplified were cloned in the vector PCR2.1 (Invitrogen) and sequenced.

Analogously to what is described in Example 1 for the genes encoding the GPCR receptors of *Spodoptera littoralis,* the cDNAs of *M. incognita* were transferred into the plasmid pENTER and subsequently into the binary vector for plants PH7GW1WG2.

The trangenic lines expressing dsRNA corresponding to MiNPR1-like of *M. incognita* were used for root infection tests.

### Root infection experiments

Two weeks old tobacco plants were trasferred into 16 cm-pots containing sterile soil mixed with around 5-6 *M. incognita* galls, previously excised from the roots of other infected plants. After 6 weeks, the soil was removed and the plant roots analyzed for the presence of galls. It was measured the amount of total biomass infected of the transgenic plants and the values compared to those of WT control uninfected plants.

### RESULTS

As described above, tobacco plants belonging to 3 different transgenic lines expressing dsRNA of NPR1-like were infected with *M. incognita* worms. After 3 weeks, it was estimated the percentage of infected roots of the transgenic plants and compared to that of untransformed control plants (WT). As shown in Figure 6, the roots of the transgenic plants showed a reduction in the infection level compared to WT plants. This result indicates that the transgenic plants, expressing the dsRNA of *M*. *incognita* NPR1-like gene, are more resistant to the nematode infection, confirming that the technology here proposed find applications also in the protection of plants against nematode attacks.

### BIBLIOGRAPHY

- Aguilar R, Maestro JL, Vilaplana L, Pascual N, Piulachs MD, Bellés X (2003). Regul Pept. Oct 15; 115(3): 171-7.
- Baum JA, Bogaert T, Clinton W, Heck GH, Feldman P, Ilagan O, Johnson S, Plaetinck G, Munyikwa T, Pleau M, vaughn TY and Roberts J (2007). Nature Biotech, 25 (11): 1322-1326.
- Brantl, S. (2002) Biochimica et Biophysica Acta. 1575: 15-25.
- Farooqui T (2007). Neurochem Res. 32: 1511-1529.
- Hamilton, A.J. and Baulcombe, D.C. (1999). Science 286: 950-952.
- Hammond, S.M. Boettcher, S., Caudy, A., Kobayashi, R. and Hannon, G. (2001). Science 293: 1146-1150.
- Johnson EC, Bohn LM and Taghert PH (2004). Journal of Exper Biol, 207: 743-748.
- Kusaba, M., Miyahara, K., Lida, S., Fukuoka, H., Takario, T., Sassa, H., Nishimura, M., Nishio, T. (2003) Plant Cell 15: 1455-1467.
- Li L, Wright SJ, Krystofova S, Park G, Borkovich KA (2007). Annu Rev Microbial., 61: 423-452.
- Meyering-Vos M, Merz S, Sertkol M, Hoffmann KH (2006). Insect Biochem Mol Biol. Jun;36(6): 492-504.
- Neuhaus EM, Gisselmann G, Zhang W, Dooley R, Stortkuhl K Hatt H (2005). Nature Neurosc, 8 (1): 15-17.
- Nishi S, Hsu SY, Zell K and Hsueh JW (2000). Endocrinology, 141 (11): 4081-4090.
- Ogita, S., H., Uefuji, Y.,Yamaguchi, N. Koizumi and Sano, H. (2003). Nature 423: 823.
- Rafaeli A, Bober R, Becker L, Choi MY, Fuerst EJ and Jurenka R (2007). Insect Molec Biol, 16 (3): 287-293.
- Staubli F, Jorgensen TJD, Cazzamali G, Williamson M, Lenz C, Sondergaard L, Roepstorff P and Grimmelikhuijzen JP (2002). PNAS, 99 (6): 3446-3451.
- Tomoyasu, Y. and Denell, R. E. (2004). Dev. Genes Evol. 214: 575-578.
- Weaver RJ, Freeman ZA, Pickering MG, Edwards JP. (1994). Comp. Biochem. Physiol. C 107: 119-27.

## Claims

1. Method for the preparation of transgenic plants resistant to the attack of phytopathogens like *Spodoptera littoralis* and/or *Meloidogyne incognita* by RNA interference comprising the following steps:
a) isolation of the cDNA nucleotide sequence encoding a G protein coupled receptor (GPCR), or a portion thereof, whose function is vital for the phytopathogen, wherein said cDNA nucleotide sequence encodes for a GPCR receptor selected between AlstCR of SEQ ID NO:2, DHR of SEQ ID NO:3 or Oct/TyrR of SEQ ID NO:6 when said insect is *Spodoptera littoralis* and wherein said cDNA nucleotide sequence encodes for a GPCR receptor selected between serotonin-like receptor of SEQ ID NO: 9 or neuropeptide receptor of SEQ ID NO:11 when said phytopathogen is *Meloidogyne incognita;*
b) construction of an expression vector comprising the cDNA nucleotide sequence encoding a GPCR receptor or a portion thereof as determined by step a), flanked by two specific recombination sites;
c) recombination reaction of the expression vector of step b) with a binary vector comprising a constitutive or tissue-specific promoter, the same specific recombination sites and an intronic sequence to obtain a recombinant plasmid;
d) transfer of said recombinant plasmid into competent cells of *Agrobacterium tumefaciens* and transformation of the plant;
e) growing the plant and obtaining the constitutive or tissue-specific expression of dsRNA in said plant.

2. Method according to claim 1, wherein when the dsRNA expression is tissue-specific, said tissue is selected from the group consisting in phylloplane, root system, trunk, buds, flowers, fruits.

3. Method according to any of claims 1-2, wherein steps a)-c) may be repeated in parallel using in each step a cDNA nucleotide sequence encoding a GPCR receptor which is vital for *Spodoptera littoralis* and/or *Meloidogyne incognita* for obtaining one or more recombinant plasmids and d) co-transforming the same plant.

4. Method according to any of the preceding claims, wherein said plant belongs to the family of Solanaceae, preferably tobacco.

5. Method according to any of the claims 1-4 for the preventive treatment of plants to make them resistant to the attack of *Spodoptera littoralis* and/or *Meloidogyne incognita,* further comprising a step for treatment of the plants thus obtained with fertilizers, biostimulating agents, fungicides, synthetic or natural nematocides, herbicides or insecticides.

6. Transgenic plant resistant to *Spodoptera littoralis* **characterized in that** it is a tobacco plant and comprises a dsRNA wherein the antisense strand is complementary to the mRNA encoding for SEQ ID NO:2 and/or SEQ ID NO:3.

7. Transgenic plant resistant to *Meloidogyne incognita* **characterized in that** it is a tobacco plant and comprises a dsRNA wherein the antisense strand is complementary to the mRNA encoding for SEQ ID NO:9 and/or SEQ ID NO:11.

## Patentansprüche

1. Verfahren zur Herstellung von transgenen Pflanzen, die gegen Pflanzenkrankheitserreger wie *Spodoptera littoralis* und/oder *Meloidogyne incognita* widerstandsfähig sind durch RNA-Interferenz, umfassend folgende Schritte:
a) Isolierung der cDNA-Nukleotidsequenz, die einen G-Protein-gekoppelten Rezeptor (GPCR) oder einen Teil desselben kodiert, dessen Funktion lebensnotwendig für den Pflanzenkrankheitserreger ist, wobei
die cDNA-Nukleotidsequenz für einen GPCR-Rezeptor kodiert, der aus AlstCR von SEQ ID NO:2, DHR von SEQ ID NO:3 oder Oct/TyrR von SEQ ID NO:6 ausgewählt wird, wenn das Insekt *Spodoptera littoralis* ist, und wobei die cDNA-Nukleotidsequenz für einen GPCR-Repzeptor kodiert, der aus dem Serotonin-ähnlichen Rezeptor von SEQ ID NO: 9 oder dem Neuropeptid-Rezeptor von SEQ ID NO:11 ausgewählt wird, wenn der Pflanzenkrankheitserreger *Meloidogyne incognita* ist;
b) Aufbau eines Expressionsvektors, umfassend die cDNA-Nukleotidsequenz, die einen GPCR-Rezeptor oder einen Teil desselben, wie in Schritt a) hergestellt, kodiert, flankiert durch zwei spezifische Rekombinationsstellen;
c) Rekombinationsreaktion des Expressionsvektors aus Schritt b) mit einem binären Vektor, umfassend einen konstitutiven oder gewebespezifischen Promotor, dieselben spezifischen Rekombinationsstellen und eine Intron-Sequenz, um ein rekombinantes Plasmid zu erhalten;
d) Übertragen des rekombinanten Plasmids in kompetente Zellen von *Agrobacterium tumefaciens* und Transformation der Pflanze;
e) Züchten der Pflanze und Erhalten der konstitutiven oder gewebespezifischen Expression der dsRNA in der Pflanze.

2. Verfahren nach Anspruch 1, wobei dann, wenn die dsRNA-Expression gewebespezifisch ist, das Gewebe aus der Gruppe bestehend aus Blättern, Wurzelsystem, Stängeln, Knospen, Blüten und Früchten gewählt wird.

3. Verfahren nach einem beliebigen der Ansprüche 1 bis 2, wobei die Schritte a)- c) parallel wiederholt werden können, wobei in jedem Schritt eine cDNA-Nukleotidsequenz verwendet wird, die einen GPCR-Rezeptor kodiert, der lebensnotwendig für *Spodoptera littoralis* und/oder *Meloidogyne incognita* ist, um ein oder mehrere rekombinante Plasmide zu erhalten und d) dieselbe Pflanze zu ko-transformieren.

4. Verfahren nach einem beliebigen der vorstehenden Ansprüche, wobei die Pflanze zur Familie der Solanaceae, vorzugsweise Tabak, gehört.

5. Verfahren nach einem beliebigen der Ansprüche 1 bis 4 für die Vorbehandlung von Pflanzen, um sie resistent zu schaffen gegen *Spodoptera littoralis* und/oder *Meloidogyne incognita,* ferner umfassend einen Schritt für die Behandlung der Pflanzen, die mittels Düngern, biostimulierenden Wirkstoffen, Fungiziden, synthetischen oder natürlichen Nematiziden, Herbiziden oder Insektiziden erzielt wird.

6. Transgene Pflanze, die gegen *Spodoptera littoralis* resistent ist, **dadurch gekennzeichnet, dass** es eine Tabakpflanze ist und sie eine dsRNA umfasst, wobei der Antisense-Strang komplementär zur mRNA ist, die für SEQ ID NO:2 und/oder SEQ ID NO:3 kodiert.

7. Transgene Pflanze, die gegen *Meloidogyne incognita* resistent ist, **dadurch gekennzeichnet, dass** es eine Tabakpflanze ist und sie eine dsRNA umfasst, wobei der Antisense-Strang komplementär zur mRNA ist, die für SEQ ID NO:9 und/oder für SEQ ID NO:11 kodiert.

## Revendications

1. Procédé pour la préparation de plantes transgéniques résistantes à l'attaque de phytopathogènes tels que *Spodoptera littoralis* et/ou *Meloidogyne incognita* par interférence ARN comprenant les étapes suivantes :
a) l'isolation de la séquence nucléotidique d'ADNc codant un récepteur couplé aux protéines G (RCPG), ou une portion de celui-ci, dont la fonction est vitale pour le phytopathogène, dans lequel ladite séquence nucléotidique d'ADNc code pour un récepteur RCPG sélectionné entre AlstCR de SEQ ID NO:2, DHR de SEQ ID NO:3 ou Oct/TyrR de SEQ ID NO:6 quand ledit insecte est *Spodoptera littoralis* et dans lequel ladite séquence nucléotidique d'ADNc code pour un récepteur RCPG sélectionné entre un récepteur type sérotonine de SEQ ID NO:9 ou un récepteur neuropeptide de SEQ ID NO:11 quand ledit phytopathogène est *Meloidogyne incognita ;*
b) la construction d'un vecteur d'expression comprenant la séquence nucléotidique d'ADNc codant un récepteur RCPG ou une portion de celui-ci comme déterminé par l'étape a), encadré par deux sites de recombinaison spécifiques ;
c) la réaction de recombinaison du vecteur d'expression de l'étape b) avec un vecteur binaire comprenant un promoteur constitutif ou spécifique du tissu, les mêmes sites de recombinaison spécifiques et une séquence intronique pour obtenir un plasmide recombinant;
d) le transfert dudit plasmide recombinant dans des cellules compétentes d'*Agrobacterium tumefaciens* et la transformation de la plante;
e) la croissance de la plante et l'obtention de l'expression constitutive ou spécifique du tissu d'ARNdb dans ladite plante.

2. Procédé selon la revendication 1, dans lequel, quand l'expression d'ARNdb est spécifique du tissu, ledit tissu est sélectionné à partir du groupe comprenant le phylloplan, le système racinaire, le tronc, les bourgeons, les fleurs, les fruits.

3. Procédé selon l'une quelconque des revendications 1 à 2, dans lequel les étapes a)-c) peuvent être répétées en parallèle en utilisant dans chaque étape une séquence nucléotidique d'ADNc codant un récepteur RCPG qui est vital pour *Spodoptera littoralis* et/ou *Meloidogyne incognita* pour obtenir un ou plusieurs plasmides recombinants et d) la co-transformation de la même plante.

4. Procédé selon l'une quelconque des revendications précédentes, dans lequel ladite plante appartient à la famille des Solanaceae, de préférence le tabac.

5. Procédé selon l'une quelconque des revendications 1 à 4 pour le traitement préventif de plantes pour les rendre résistantes à l'attaque de *Spodoptera littoralis* et/ou *Meloidogyne incognita,* comprenant en outre une étape pour le traitement des plantes ainsi obtenues avec des engrais, agents biostimulants, fongicides, nématocides synthétiques ou naturels, herbicides ou insecticides.

6. Plante transgénique résistante à la *Spodoptera littoralis* **caractérisée en ce qu'**elle est une plante de tabac et comprend un ARNdb dans lequel le brin antisens est complémentaire à l'ARNm codant pour SEQ ID NO:2 et/ou SEQ ID NO:3.

7. Plante transgénique résistante à la *Meloidogyne incognita*, **caractérisée en ce qu'**elle est une plante de tabac et comprend un ARNdb dans lequel le brin antisens est complémentaire à l'ARNm codant pour SEQ ID NO:9 et/ou SEQ ID NO:11.


### SEQUENCE LISTING

<110> Arterra Bioscience SRL
<120> METHOD TO OBTAIN TRANSGENIC PLANTS RESISTANT TO PHYTOPATHOGEN ATTACK BASED ON RNA INTERFERENCE (RNAi)
<130> PCT97529
<150> MI2008A1305
   <151> 2008-07-17
<160> 101
<170> PatentIn version 3.2
<210> 1
   <211> 1086
   <212> DNA
   <213> Spodoptera littoralis
<220>
   <221> misc_feature
   <222> (1)..(1086)
   <223> Allatostatin A receptor encoding cDNA
<400> 1
   atggcgtcga ctgaagacga atttaggagc atatgcctta acttaaccag tgaggaggct     60
   atgcttggag gatgtaacta cagcagcgaa tatgaaaatg gagaactgtt ggagaaggtc    120
   gtctcgaggg ttgtgcccat cttctttggc ttcataggca tcgttggatt agttggcaat    180
   gctttggtcg tcttagtggt cgctgccaac cctggcatgc ggtccactac caacctgctc    240
   atcataaact tggctgtcgc tgacttgctc tttgtgatct tctgcgttcc attcactgct    300
   accgactatg tcatgccacg atggcccttc ggagactggt ggtgcaaggt tgtccagtac    360
   ttcattgtag tcactgctca tgcgtccgtc tacactctag tactcatgtc tctagacagg    420
   ttcatggctg tagtgcatcc catagcttcc atgtctatca gaacagagaa gaacgcatta    480
   cttgctatag cttgcatatg ggtggtgata ttaaccacag ctattcccgt gggcatctgc    540
   cacggggagc gggaatactc ctacttccat agaaaccatt cctcctgcgt gttccttgag    600
   gagcaaggat atagcaagct agggttccag atgtctttct ttttgtcttc ctatgtgatt     660
   cccttagctt tgattagtgt attgtatatg tgtatgctga cgaggttgtg gaagagtgct    720
   ccaggaggga gggtgtctgc ggagagtcgg agggggagga agaaggtgac gagaatggtt    780
   gtggtcgtag tggtggtgtt cgcagtgtgc tggtgcccaa tacagataat cctgttggta    840
   aaggcgctgg acaagtacca catcacatac ttcacagtca ctgcgcagat agtgtcgcat    900
   gtactagcct acatgaacag ctgcgtcaac ccagtgctgt acgccttcct ctctgagaac    960
   ttcagggtcg ccttcaggaa ggtaatgtac tgcccgcccc cctacaatga cggcatgtca   1020
   ggccggcccc agcccaccaa gacgactcgt acaggcaatg ggaactcctg ccatgacatc   1080
   gtctga                                                              1086
<210> 2
   <211> 726
   <212> DNA
   <213> Spodoptera littoralis
<220>
   <221> misc_feature
   <222> (1)..(726)
   <223> Allatostatin C receptor encoding cDNA
<400> 2
   tgctttctta tcggcatccc attcctgatc acaacgatgt ctcttcgtgc gtggccgttt 60
   ggacgcttca tgtgtaaggc ctacatgata tctaccggaa tcaaccagtt caccagctct 120
   atattcttgt gtataatgag tgcggatcga tacatagccg tgtgtcatcc gatagccgcg 180
   ccgcgactgc ggacgccatt cgtctctaga gtggtgtcag ctgcagcatg gacagcgtca 240
   gctttggtga tgacgcctat atttatgtac acgacactca taccaacacc ccatggtctg 300
   tcgtgcaata tagtgtggcc cgagcaagag ttcaataaag gccagacgtc cttcactctg 360
   tactcgttcg ctctcggttt cgccgcacca ctcaccctta tcttcgtgtt ttactgtctc 420
   gtgattcgca aactgaagac tgtcggccct aagaacaaat ctaaagagaa aaagcgatca 480
   cacagaaaag taaccaaatt agtgctgact gtcattgctg tgtacgtgct gtgctggctg 540
   ccgtactggg cgttccaggt agctctcatt tactctccac cgaacgagtg cgccagccag 600
   atcactatca ccgtttttct ggtagccgcg tgcttcagtt acagtaactc tgccatgaac 660
   ccaattcttt acgcctttct ttctgacaac ttcaagaaaa gtttcctaaa ggcctgcacc 720
   tgtgcc 726
<210> 3
   <211> 1191
   <212> DNA
   <213> Spodoptera littoralis
<220>
   <221> misc_feature
   <222> (1)..(1191)
   <223> DHR receptor encoding cDNA
<400> 3
   atggcggaga agtgcctggc gcagaacgaa gggctggcgg aggaggagtt ctgccccgcg 60
   tactacgacg ggctgctgtg ctgggacgcg acgccctgga acacgctggc ggtgcagcgc 120
   tgcttcagcg agttccacgg cgtccagtac gatgacacac agaacgcatc gcggatgtgc 180
   ttggacggcg tgtggcacaa ctactcaaac tacgcgaact gcacagaacg catgaccaac 240
   gtgtcgccga ctgacgtcac cagcctcatc tacctcgccg gctactcgct cagcctcgcc 300
   gtgctttcac tcgccgtctt cgtcttcctt tatttcaagg acctgcgatg tctaaggaac 360
   acgatacata ccaacctcat gtcgacgtat attctgtctg cctgcagctg gatcttaaac 420
   ttagctttac aaaattggtc cgatgaggcc caacaagatc aaacctcgtg tacgattctg 480
   gtcatatgta tgcactactt ttacctcact aatttcttcg ggatgcttgt agaaggtttg 540
   tacctgtaca tgctcgtggt ggagacgttc accgctgaaa atattaaact gaaggtgtac 600
   actaccatag gatggggtgc gccggcggtg ttcctaacaa tatgggttat atgcaggtgc 660
   ttcgtcaaca tcgtgccttc caaccctgct gatgggcttg ccctggcagg tgaggctaag 720
   atgtgtattt ggatgtcgga gcaccaggtg gattggatcc ataaagctcc agcactcgct 780
   gggctcgccc tcaacctctt cttcctcatt aggattatgt gggtattaat cacaaaactg 840
   cggtctgcaa acacgttgga gacggagcag tatcggaaag caacgaaggc acttctagtc 900
   ctgatcccac tgctgggcat caccaacctg ctggtgctgt gtgggcccaa tgacgactcc 960
   tggttcgcac acgccttcga ctacacgcga gcactcatgc tttcaacaca gggtttcttg 1020
   gtggctttgt tctactgctt catgaataca gaagtacggc acgcaatcag ataccacgtc 1080
   gagaggtgga agactgggag gaccatcggt ggaggaagga ggagaggagc ttcctactcc 1140
   aaggactggt ctcctcggtc acgaacagaa agcatacgac tcacggtatg a 1191
<210> 4
   <211> 534
   <212> DNA
   <213> Spodoptera littoralis
<220>
   <221> misc_feature
   <222> (1)..(534)
   <223> AKHR receptor encoding cDNA
<400> 4
   gacctgatgt gccgagtcat gatgttcacc aggaccttcg gactgtacct ctccagcttc 60
   gtgcttatct gtatagctgt tgatagatac tacgccattt taaagccttt gaacgtgaca 120
   tgggaggcgc gcgtgcgcag agcgctgatt atcgcgtggg tgtgcgccgg cctcgccagc 180
   ctgccgcaga gcttcatatt ccacgtagag gagcatcctg atgttaaggg gtactaccag 240
   tgcgtgtcgt acgggtcgtt gccgacggag tgccacgagt ttgcgtactt cttggtaaac 300
   atgatcctca tgtacgtcat ccctctgctg tccacgctgt actgctcctc ggcagctttg 360
   ttggaaatca ttcggagatc taacacttct aatgataaga tgcgacggag cggcgtgggt 420
   atactcgggc gggcgcgggc tcggacactc aagatgacag tcactatagt tctagtcttt 480
   tttacatgtt ggtcacccta ctactgttat tgtctgtggt attggatcga caag 534
<210> 5
   <211> 2250
   <212> DNA
   <213> spodoptera littoralis
<220>
   <221> misc_feature
   <222> (1)..(2250)
   <223> LGR1 receptor encoding cDNA
<400> 5
   atgtattgga gattatgtat ttgggctgtt gcctttattt tactggggag cagagaagca 60
   gcatgccact cgatgagtga gagtgtgtct gtggtgctgc accagaccaa catcacacgg 120
   ctcacggcaa aggtcatcca tgctgcgggc taccatttgc atcgagttga gcatttatca 180
   ataatggatg caccaaaatt ggatcacata gtagtggacg atctgactaa gatgcctaat 240
   ctcaaatctc tgtttataac gcaagctccg cgactacaca gggtgtcgcc cctccctccg 300
   ctgcccgagc tacgcacatt catcatcaca acctccggct tggtggaagt ccccaacctg 360
   agccacgtgc acgacaagaa gaataacaac tcactcgcct atctgcaagc tatagaccta 420
   gaaggcaacc acataaagcg cctatcgccg cacgcattgc gcgtgcgcgc tgatcaagtg 480
   tccttgaact acaacttgct agaagaagtg cctgcttttg cgttcaagaa tgcgcagatt 540
   tcaaaattga gtttcagagg aaacacgaaa ctaaaacata ttgatgaaaa tgccttcgca 600
   tcgaatttgc tcctccgaca attagattta agtaacaccg ctattacctc cctgcctaca 660
   gcaggactac agaacttgga aacccttaga atagaaaaga caccttcatt gaaatacatc 720
   ccatcgattt acgaatttca gagtctccag aaagcgtacc tgacgcatca tttccactgc 780
   tgcgcgttcc acttccccga gcgacacaac ccaacgagac acaagctcta tgaaacgcaa 840
   attgctataa tgcagtcgaa atgtgcgagc gctagtcagc agaagaaacc agtagccaga 900
   agaaagaaga ggtcactggc accactcaag atcgttatgc ctcagcgtaa aaacgatact 960
   acagactcat tagaggaaga cctgtcaaca gcgactacga gcgaggagta cgacgctaac 1020
   ttgatggacg agtacttctc agactccggc agttgggagg acgaggacga ccagggagag 1080
   ttccacgaac cagtcaacaa cacgctcggg gccggcttcg cgatcatatc cgctgagtgc 1140
   gggaacttca gtattagccc acgaaaagtg gaatgctttc ccgcacctga tgcactgaat 1200
   ccgtgcgaag atgtgatggg ctggtcgtgg ctccgagcga gtgtgtggtt cgtcatcacg 1260
   gcggcggtgg tgggcaacgt tgccgtcctg ctcgtcttac ttacaaacca cacggagatt 1320
   acagtcccgc ggttccttat gtgccacctc gccttctctg acctctgcac cggcctctac 1380
   ctgtttatgt tggccgtcgt agacctcaga tcttatggag aattcttcaa ctacgcctac 1440
   gattggcagt acggggtcgg ctgcaagatc gctggctttc tatcagtatt ttctggacag 1500
   ttatcagtaa ttactttaac tatagtcaca ttggagcgct ggttcgccat aacttatgca 1560
   atatacttag aaaggaggat ctctctatca gccgcagcta agataatgat gggcggttgg 1620
   ttgtactcct tcctgatggc tggcttgccg ctggtcggcg tgtcagacta ttcgtctacg 1680
   tcaatatgtt taccggttga gagcaaggat gtgggagatg ctgtgtacca gggatcaata 1740
   tttatagtga gtgcgttggc gtgggtcacg attgtggtgt gctacgtgca gatatacagg 1800
   tcgctagggg gtggagagag ttacggaggg aggggggcgg ccgcagccgc tgagagacga 1860
   attgcgaata aaatggcact cctcatcgga accgacctgt tgtgctgggc gcctgtggcg 1920
   ttcttcggca tcacagctct agcaggagtg ccgctagtcg atgtcagtca cgggaaagtg 1980
   ttgctagtat tcttttaccc tctgaacgct tgtgcgaatc cattcttata cgccatatta 2040
   acgaaacaat accgaagaga tttgataacg ttgatagcac ggacgggtca ctgcacctgg 2100
   ttagtggaga agtacaagtt ggcggcgacc ccgcccccca ccgcgcacac taacccttct 2160
   gcgccggcgc agttgatgcc gctagtagac tccgtcacgt atcaaaggag ccagtcacag 2220
   tgtaaagaca gtagtgaggt accgctttaa 2250
<210> 6
   <211> 1437
   <212> DNA
   <213> spodoptera littoralis
<220>
   <221> misc_feature
   <222> (1)..(1437)
   <223> OCtR receptor encoding cDNA
<400> 6
   atggggcaaa cagctacaca cgtaaacgca aattacacat taattaatta tactgatgaa 60
   ataattgagg atgacagaga cgcttgtgcc gtcgcagatg accctaaata tccgagtagt 120
   ttcggtataa cacttgcggt accagaatgg gaagcaattt gtacagccat agtcctaacg 180
   cttataataa tctcaacgat agttggaaac attttggtga tcttgagtgt gtttacatac 240
   aaacccctta ggattgtcca gaacttcttc atagtatcac ttgctgtggc ggatttgaca 300
   gtagcaatat tagtattgcc actaaatgtg gcatactcca ttctaggaca atgggtattt 360
   gggatttacg tatgcaaaat gtggttaaca tgcgacataa tgtgttgcac ttcgtccatt 420
   ttaaatttat gtgcaatcgc attagacagg tattgggcga taaccgatcc tataaattat 480
   gcacaaaaga gaactttaga aagagtgatg ttaatgattg gagtagtttg ggtactttca 540
   ttagttatta gctctccgcc acttctaggg tggaatgact ggcccgacgt ctttgaacct 600
   gatacgcctt gtcgtttaac ttcccaacct ggcttcgtta tcttctcttc atctgggtcc 660
   ttctacatac cgctagttat aatgactgta gtttattttg aaatatattt ggccactaaa 720
   aaaagactta gagatcgcgc taaagccaca aagatcagca ctatttctag cggtcagaat 780
   agatacaata ataaagacga taacaatgat catgatcaag attctgttag ctcagaagcc 840
   aatcacaatg agcaccaagg cgtgacacgg ttggcatcag acaatgagaa aaagaaaaga 900
   accagaaaat tgacaccaaa gaaaaaacca aagcgacgat attggagtaa ggacgataaa 960
   tcgcaaaaca aactgataat accgattctc tctaacgata attcggtaac tgatatgggt 1020
   gataatttag aaaatagaaa cacatcatca gagagtaact cgaaagaaac tcacgaggat 1080
   gatttgattg aagttactga gcctgcgcca gtgaagagtc acaagcggcc gaagcctaac 1140
   cagcaaagtg ctgtttatca attcattgaa gaaaagcaaa ggatttcctt gacacgtgag 1200
   cgtcgagccg ccagaaccct tgggattata atgggcgtat ttgtcgtgtg ttggttgccc 1260
   ttctttgtca tataccttgt tattcccttc tgtgcgagtt gctgtttgtc caacaagttt 1320
   atcaatttca taacttggct tggctactgc aattctgcac tcaaccctct aatatacacc 1380
   atctttaaca tggactttag gagagcattc aaaaagttac tctgtatgaa accgtga 1437
<210> 7
   <211> 1419
   <212> DNA
   <213> Spodoptera littoralis
<220>
   <221> misc_feature
   <222> (1)..(1419)
   <223> or83b receptor encoding cDNA
<400> 7
   atgaccaaag tgaaggccca gggcctcgtc tccgacctga tgcccaacat caagttgatg     60
   caggcagctg gacacttcct cttcaattac catgcagaaa atggtggcat gagcggcctc    120
   cttcggaaga tctacgccag tacccacgcg attctcatca ccatacattt tgcctgtatg    180
   ggtatcaaca tggcccagta ctccgatgag gtcaacgagc tgactgctaa cactatcacc    240
   gtcctcttct tcactcacac catcatcaag ttggggttct tcgctttgaa ctccaagagt    300
   ttttacagga ctttagcagt atggaaccag tcgaacagcc accctctgtt cacggagtca    360
   gatgcccgct accatcaaat tgcgctcacc aagatgagga gactcctgta cttcatctgc    420
   ggcatgactg tcctgtcagt tgttagctgg gtcacactca cattcttcgg tgaatcagtc    480
   cgcctgatta ctagtaagga aacgaatgag acgttgacag aagtagcccc tcgattgcct    540
   ctgaaggcct ggtacccttt caatgctatg agcggcacca cgtatattat cgctttcgct    600
   ttccaggtct actggctcct cttctcaatg gctatagcaa atctgatgga tgttatgttc    660
   tgctcctggc tgatcttcgc ttgtgaacag ctgcagcacc tgaaggccat catgaaacct    720
   ctcatggaac tcagtgcttc actggacacg tatcggccta atactgctga gctgttccgt    780
   gcttcttcca ctgagaaatc agaaaagatt cctgatacag tcgacatgga catccgcggc    840
   atatattcca cacagcaaga tttcgggatg acgttgcgag gagctggagg tagactgcag    900
   gcctttggac agcagaacaa caaccctaat ggcttgactc cgaagcagga gatgctggcg    960
   aggtctgcta ttaagtattg ggtggagagg cacaaacacg tcgtcagatt ggtagcatcg   1020
   ataggagaca cgtatggtac tgccctgctg ttccacatgt tggtgtcaac tataacactc   1080
   actctattgg cttaccaggc tactaagatc aatggaatta atgtctacgc gttcagtact   1140
   attggctacc tcagctatac cctgggacag gtgttccatt tctgtatctt cgggaacagg   1200
   cttattgaag agagttcctc agtgatggaa gccgcctact cctgtcagtg gtacgacggc   1260
   tccgaggaag caaagacctt cgtccagatc gtgtgccagc aatgccagaa agccatgagc   1320
   atctccgggg ccaagttctt cacggtgtca cttgatcttt ttgcttcggt acttggtgca   1380
   gtagttactt acttcatggt gttggtacaa ctcaagtaa                           1419
<210> 8
   <211> 1053
   <212> DNA
   <213> Spodoptera littoralis
<220>
   <221> misc_feature
   <222> (1)..(1053)
   <223> PBANR receptor encoding cDNA
<400> 8
   atgacattgt cagcgccccc gatcgacgag tttgaggacc cgttcgtcgt gatgaacacg     60
   acgaatgtgt cgagtcaccc ggccgcctat gacgagccat acacgctcga tctggtggtg    120
   ccactcaccg tcacctacgt ggtgatattc gtagccggca tcctggggaa caccagcacc    180
   tgtgtggtca ttgcgaggaa tcgctctatg cacaccgcta caaactttta cctcttcagt    240
   ttagcaattt cggatctcat attgttagta tgtggtctac cgtttgaggt gcacagatta    300
   tggaacccgg acacgtaccc gttgggcgag gctcactgca tcgccattgg gttagcgtcg    360
   gagacttcag ctaacgcgac agtgttgaca ataacagcgt ttacggtgga gcggtacatt    420
   gcgatatgta gacctttcat gtcgcacacc atgtcgaagc tgtcacgagc tgtgcggttt    480
   ataatagcta tttgggtgtt tgcgttgtgt actgcggtac cacaagcgat gcagttcggt    540
   atagtgtcgt atgtggataa tggtcagaat gtgagcgcgt gcacagtgaa gggtgtgggt    600
   gttcatcaag tgttcgttat atctagtttc gtgttcttcg tggtgcctat gtcgatgata    660
   tcagtgttgt acgcgttgat tggtattaag ttgcggatct ctcgagtgtt gcatccagtg    720
   aagaagctct cggtggacag caacgaaaga gcgagtggac agatgcagta caggaatgga    780
   gcatcacaga ggagagtcat tagaatgctc gttgcggtgg cgctgtcatt cttcatttgc    840
   tgggcgccct tccacgtgca aaggttgctg gccatctacg ggaaaagttt agaacaccct   900
   tctgatactt tctacttggt ctacatcgtg ctgacgtttt tgtcgggagt gctatatttc    960
   ctgtcaaccg ccatcaaccc gttcctgtac aacatcatgt cgaataagtt tagaaacgcc   1020
   ttcaaggtga gtttttgtta cattcatgat tga                                1053
<210> 9
   <211> 1656
   <212> DNA
   <213> Meloidogyne incognita
<220>
   <221> misc_feature
   <222> (1)..(1656)
   <223> serotonin-like receptor encoding cDNA
<400> 9
<210> 10
   <211> 1601
   <212> DNA
   <213> Meloidogyne incognita
<220>
   <221> misc_feature
   <222> (1)..(1601)
   <223> Dopamine-like receptor encoding cDNA
<400> 10
   atgttgccct ggtggctacc tcttttaatt tttgtcccca tcttttctgt attaatttcc      60
   cttgcctttt ctggcaacct tctcgtaatc gcagcaatat tttacgacag aaaattgcgt     120
   agacaaccag aaaatttatt tttagtttcc ttagctttgt cagacttgtt tgttgctgga     180
   atggttatgg ttttagcggc agcaaacgat ttaattggtt tttggccttt tggggctgct     240
   ttttgtcaat tttggatttg tttggatatt gcttgtagta ctgcttctat tttaaattta     300
   tgtgcaatag ctctcgatcg ttttattcac attagcaggc caatgcgtta tgttagattt     360
   gttggaagaa gggttatttg ttgttctgtt tgtgctattt ggataatttc tacagctgtt     420
   ggaacggccc aaatagcttt tggggctact aatggattgg agatatattc tgttatattt     480
   aatggagact attttggaga gattaatgag actgataaat ttagattcat cgaacaacaa     540
   catacactag ttcaatgcca attacaatta tctccttttt atgccgtttt ttcttcctta     600
   cttagcttct tcctaccagc tactttgatg cttttctttt actacaggct ttatttatat     660
   gctcggcatc atgctcgttc tatacaatca caacttaaac aggctacttc acttttgatt     720
   tacaattagc ctcagataga gtacgtcaag tagttgtacg tccttctagt ggatttttat     780
   ccccctcttc aatagccctt cactacgatt ctcgaagatt tagtggaaaa tcctcctttg     840
   atggagaatc acttcaaaaa gccgagttta gccaaataaa cgttgctgtg aataaatcac     900
   cagaaaatca ttctatcgac gctacaatcc aacaaggaac gcctgtactt agggctacat     960
   tgagacaatt aagaaggaca gaaagcaata atagtagcag ctttagcttt gtttcgagaa    1020
   atggattggg tgttaatagc agaagggttg gttgtggtgg tggtaatggt ggggagagaa    1080
   gaaagagttg ttccccactt ccttctccaa caacaaaaag aggttttaaa tcttgtgggt    1140
   ggtctaataa taataatggc aatggagggc atcgttcttc tgacaaaaaa gcacgtgtta    1200
   ctcttggtgt tattatgggt acttttctaa tatgttggtt acccttcttt attgtcaatg    1260
   ttctacgccc tttctggcct gaaatttttc ctccactact ttttcagaca gtttcctggc    1320
   taggttatgc caattcttca gtaaatccag taatttatgg aatatttaat cgagactttc    1380
   gacgtgcctt tagccgtata atgaacaaat taattcattg tatagacgag gaaagaaggg    1440
   gagaaaatag tcgaggaggg acatttagaa ctagaacaga aagtgaatct tttaataaat    1500
   ttaataaaag tttaaataat gcaagtgaac tacaacaaca acaaaaatta atttttaaac    1560
   aaaaaaagac aaataaaaaa ttgagatttt tatgttttta a                        1601
<210> 11
   <211> 1484
   <212> DNA
   <213> Meloidogyne incognita
<220>
   <221> misc_feature
   <222> (1)..(1484)
   <223> Neuropeptide receptor encoding cDNA
<400> 11
   atggaagcat ctacaatgga attacttcca cctcccacca tattatttaa tgtatcatca      60
   gcgccttctg ttatttgtga ggatatgaat gcttatttat ggaacactcg tcgggatttg     120
   actacatgtc ctttcgttat ggctctattt gcttccttaa ttcatcaatc attttactgg     180
   gtattatcgg aaactcttgt gtgattatgg ctattgccag aattaaatca cttcaaactg     240
   tgcctaatat gtttattttc tcgttgagtt gttcagatat acttgtttgc tttatttctg     300
   ctactataac tccaatcgct gccttcaaaa aggattggat ttttggccaa ttcttatgct     360
   cttttgcacc ttttgttgcg ggagggagtt tgtgtttctc tacattcact ttggcggcta     420
   tttccgtgga ccgttttctt ctaatacttt ttcccacgag aaaggctttc tcgcatacac     480
   aagctttatt tataattctt gtcacgtttc tgttggctag cgggtttagt ttaccaatgt     540
   tgttcatgca aaagttaaaa ccagtaactc attattgtgg ccgattttgt ttcgaggatt     600
   ggggcgagat gattggaata agacgaatct atggaacttt attgctgacc gtccaattca     660
   ttattccgtt agcattgatc actttctgtt acacagcaat ttcattccga cttggaaaaa     720
   gcgtgaatct acgtactagg aagaaatgtg aatggcaaat gccgattagt gctcagcgaa     780
   acgcggccac aaagcgaaga caacgaacta accgaatgtt tattgcaatg gtaattgcct     840
   tctcagtgag ctggatatgg tcggttctat ataatatgtt acgagactat gacggtcttc     900
   caaagtttgt acatgatcaa gaattctttt ttggaatatt aacacactgt attgcaatga     960
   gctccacggt gtggaatccg atcttatatg ccttattaaa tctacaacta agggcagcct    1020
   ttctacaact tgtgccacaa tgtattaagc attctctgac ttgcacttgt agtcctaata    1080
   aaggaagatt aagcggtcga tcagttgctc gatgtgccta ccttgaagag agccggcttc    1140
   ttggttcctc agttcttcgc ggaacaaccg gacaagtaca gagaagagaa acgaattatg    1200
   aaaataaaaa ctgttttaat tgtttgattg gctcctctag atccgttgaa tcttcttcaa    1260
   acgaaacaca gaagcaaacg ttttctgtta aatgtgaagg aaataaagga gatgctaaaa    1320
   ttttgtcaca taatcaccat cggcatcaat tagaaagaat aacagcggac ggtggatttg    1380
   acgagcaaac atggacagag caactgagga aggactctga agatgatgcc ttagatgatt    1440
   gtaatgatga tttgagagaa agagatacag atgagaggat atga                     1484
<210> 12
   <211> 57
   <212> DNA
   <213> Artificial
<220>
   <223> AlstAR degenerate 1Forward Primer
<400> 12
   atgacgacgt atgcacgtac agctacagct aatctctagc ttctagctat tcaagtc 57
<210> 13
   <211> 51
   <212> DNA
   <213> Artificial
<220>
   <223> AlstAR degenerate 2Forward primer
<400> 13
   gtagctccag cttttcacag ctgcagctac agctgatcta tcgtagctat g 51
<210> 14
   <211> 52
   <212> DNA
   <213> Artificial
<220>
   <223> AlstAR degenerate 1Reverse primer
<400> 14
   gtagctacag ctacgagcta tggtagctgt agctgtagct gtagctgtag ct 52
<210> 15
   <211> 51
   <212> DNA
   <213> Artificial
<220>
   <223> AlstAR degenerate 2Reverse primer
<400> 15
   tggatsagct tgtcgtagct aatcccatcg gtatgcactt ctatcgcagc t 51
<210> 16
   <211> 20
   <212> DNA
   <213> Artificial
<220>
   <223> 3'RACE amplification oligonucleotide 1s
<400> 16
   gcatcccata gcttccatgt 20
<210> 17
   <211> 31
   <212> DNA
   <213> Artificial
<220>
   <223> 3'RACE amplification oligonucleotide 2s
<400> 17
   ggtgatatta accacagcta ttcccgtggg c 31
<210> 18
   <211> 31
   <212> DNA
   <213> Artificial
<220>
   <223> 3'RACE amplification oligonucleotide 3s
<400> 18
   gtatgctgac gaggttgtgg aagagtgctc c 31
<210> 19
   <211> 21
   <212> DNA
   <213> Artificial
<220>
   <223> 3'RACE amplification oligonucleotide 4s
<400> 19
   aaggtgacga gaatggttgt g 21
<210> 20
   <211> 21
   <212> DNA
   <213> Artificial
<220>
   <223> 3'RACE amplification oligonucleotide 5s
<400> 20
   gcgcagatag tgtcgcatgt a 21
<210> 21
   <211> 31
   <212> DNA
   <213> Artificial
<220>
   <223> 5'RACE amplification oligonucleotide 1as
<400> 21
   agacgactct tccacaacct cgtcagcata c 31
<210> 22
   <211> 20
   <212> DNA
   <213> Artificial
<220>
   <223> 5'RACE amplification oligonucleotide 2as
<400> 22
   gttaatatca ccacccatat 20
<210> 23
   <211> 31
   <212> DNA
   <213> Artificial
<220>
   <223> 5'RACE amplification oligonucleotide 3as
<400> 23
   gcccacggga atagctgtgg ttaatatcac c 31
<210> 24
   <211> 22
   <212> DNA
   <213> Artificial
<220>
   <223> oligo SL1
<400> 24
   ggtttaatta cccaactttg ag 22
<210> 25
   <211> 41
   <212> DNA
   <213> Artificial
<220>
   <223> oligo(dT) SL1
<400> 25
   ggtttaatta cccaactttg agtttttttt ttttttttac g 41
<210> 26
   <211> 18
   <212> DNA
   <213> Artificial
<220>
   <223> AlstAR gene amplification primer
<400> 26
   atggcgtcga ctgaagac- 18
<210> 27
   <211> 18
   <212> DNA
   <213> Artificial
<220>
   <223> AlstAR amplification primer
<400> 27
   tcagacgatg tcatggca 18
<210> 28
   <211> 22
   <212> DNA
   <213> Artificial
<220>
   <223> or83b Forward primer
<400> 28
   atgaccaaag gtgaaggccc ag 22
<210> 29
   <211> 21
   <212> DNA
   <213> Artificial
<220>
   <223> Or83b Reverse primer
<400> 29
   gtgttggtac aactcaagta a 21
<210> 30
   <211> 46
   <212> DNA
   <213> Artificial
<220>
   <223> DHR Forward primer
<400> 30
   tttctctatc gtatctttca aaggaatcgt ctatgacgat cgtgtc 46
<210> 31
   <211> 44
   <212> DNA
   <213> Artificial
<220>
   <223> DHR Reverse primer
<400> 31
   aagtcttatc ggtatcgata tcgaagatcg acccacatat cgat 44
<210> 32
   <211> 27
   <212> DNA
   <213> Artificial
<220>
   <223> 3'RACE DHR amplification oligonucleotide 1s
<400> 32
   aacctcatgt cgacgtatat tctgtct 27
<210> 33
   <211> 27
   <212> DNA
   <213> Artificial
<220>
   <223> 3'RACE DHR amplification oligonucleotide 2s
<400> 33
   atgcttgtag aaggtttgta cctgtac 27
<210> 34
   <211> 27
   <212> DNA
   <213> Artificial
<220>
   <223> 3'RACE DHR amplification oligonucleotide 3s
<400> 34
   tgggttatat gcaggtgctt cgtcaac 27
<210> 35
   <211> 27
   <212> DNA
   <213> Artificial
<220>
   <223> 5'RACE DHR amplification oligonucleotide 1as
<400> 35
   ggatggggtg cgccggcggt gttccta 27
<210> 36
   <211> 27
   <212> DNA
   <213> Artificial
<220>
   <223> 5'RACE DHR amplification primer 2as
<400> 36
   catacatatg accagaatcg tacacga 27
<210> 37
   <211> 27
   <212> DNA
   <213> Artificial
<220>
   <223> 5'RACE DHR amplification oligonucleotide 3as
<400> 37
   ggcgaggtag atgaggctgg tgacgtc 27
<210> 38
   <211> 21
   <212> DNA
   <213> Artificial
<220>
   <223> DHR Forward amplification primer
<400> 38
   atggcggaga agtgcctggc g 21
<210> 39
   <211> 21
   <212> DNA
   <213> Artificial
<220>
   <223> DHR Reverse amplification primer
<400> 39
   tcataccgtg agtcgtatgc t 21
<210> 40
   <211> 53
   <212> DNA
   <213> Artificial
<220>
   <223> LGR1 degenerate forward primer
<400> 40
   gcatgctatc tctagctaca gctcatcgca gctagctttt ccatctgtct gtc 53
<210> 41
   <211> 46
   <212> DNA
   <213> Artificial
<220>
   <223> LGR1 degenerate reverse primer
<400> 41
   tcagctatca gctggagcta agagcaagct atagctgcat agctgt 46
<210> 42
   <211> 18
   <212> DNA
   <213> Artificial
<220>
   <223> 3'RACE LGR1 amplification oligonucleotide 1s
<400> 42
   tgatgaaaat gccttcgc 18
<210> 43
   <211> 20
   <212> DNA
   <213> Artificial
<220>
   <223> 3'RACE LGR1 amplification oligonucleotide 2s
<400> 43
   cacgaaccag tcaacaacac 20
<210> 44
   <211> 27
   <212> DNA
   <213> Artificial
<220>
   <223> 3'RACE LGR1 amplification oligonucleotide 3s
<400> 44
   gaaagcgtac ctgacgcatc atttcca 27
<210> 45
   <211> 27
   <212> DNA
   <213> Artificial
<220>
   <223> 3'RACE LGR1 amplification oligonucleotide 4s
<400> 45
   gttatcagta attactttaa ctatagt 27
<210> 46
   <211> 21
   <212> DNA
   <213> Artificial
<220>
   <223> 5'RACE LGR1 amplification oligonucleotide 1as
<400> 46
   actgccggag tctgagaagt a 21
<210> 47
   <211> 20
   <212> DNA
   <213> Artificial
<220>
   <223> 5'RACE LGR1 amplification oligonucleotide 2as
<400> 47
   gtactcctcg ctcgtagtcg 20
<210> 48
   <211> 20
   <212> DNA
   <213> Artificial
<220>
   <223> 5'RACE LGR1 amplification oligonucleotide 3as
<400> 48
   catttcgact gcattatagc 20
<210> 49
   <211> 21
   <212> DNA
   <213> Artificial
<220>
   <223> 5'RACE LGR1 amplification oligonucleotide 4as
<400> 49
   gccttctagg tctatagctt g 21
<210> 50
   <211> 27
   <212> DNA
   <213> Artificial
<220>
   <223> LGR1 amplification forward primer
<400> 50
   atgtattgga gattatgtat ttgggct 27
<210> 51
   <211> 27
   <212> DNA
   <213> Artificial
<220>
   <223> LGR1 amplification reverse primer
<400> 51
   ttaaagcggt acctcactac tgtcttt 27
<210> 52
   <211> 26
   <212> DNA
   <213> Artificial
<220>
   <223> Oct/TyrR degenerate forward primer
<400> 52
   ccagaatggg aaggcatatt ctgcac 26
<210> 53
   <211> 27
   <212> DNA
   <213> Artificial
<220>
   <223> Oct/TyrR degenerate reverse primer
<400> 53
   acagcccatt attgatagcc tcagagg 27
<210> 54
   <211> 18
   <212> DNA
   <213> Artificial
<220>
   <223> 3'RACE Oct/TyrR amplification oligonucleotide 1s
<400> 54
   ccagaaaatt gacaccaa 18
<210> 55
   <211> 20
   <212> DNA
   <213> Artificial
<220>
   <223> 3'RACE Oct/TyrR amplification oligonucleotide 2s
<400> 55
   gagagtaact cgaaagaaac 20
<210> 56
   <211> 24
   <212> DNA
   <213> Artificial
<220>
   <223> 3'RACE Oct/TyrR amplification oligonucleotide 3s
<400> 56
   tgctgtttat caattcattg aaga 24
<210> 57
   <211> 22
   <212> DNA
   <213> Artificial
<220>
   <223> 5'RACE Oct/TyrR amplification oligonucleotide 1as
<400> 57
   agttcttttt ttagtggcca aa 22
<210> 58
   <211> 19
   <212> DNA
   <213> Artificial
<220>
   <223> 5'RACE Oct/TyrR amplification oligonucleotide 2as
<400> 58
   gacaaggcgt atcaggttc 19
<210> 59
   <211> 26
   <212> DNA
   <213> Artificial
<220>
   <223> 5'RACE Oct/TyrR amplification oligonucleotide 3as
<400> 59
   accctagaag tggcggagag ctaata 26
<210> 60
   <211> 21
   <212> DNA
   <213> Artificial
<220>
   <223> oct/TyrR amplification forward primer
<400> 60
   atggggcaaa cagctacaca c 21
<210> 61
   <211> 18
   <212> DNA
   <213> Artificial
<220>
   <223> Oct/TyrR amplification reverse primer
<400> 61
   ctctgtatga aaccgtga 18
<210> 62
   <211> 24
   <212> DNA
   <213> Artificial
<220>
   <223> PBANR amplification forward primer
<400> 62
   atgacattgt cagcgccccc gatc 24
<210> 63
   <211> 18
   <212> DNA
   <213> Artificial
<220>
   <223> PBANR amplification reverse primer
<400> 63
   tcaatcatga atgtaaca 18
<210> 64
   <211> 30
   <212> DNA
   <213> Artificial
<220>
   <223> AlstCR degenerate forward primer
<400> 64
   gaagtgtctt tctctagcta tatcggatgc 30
<210> 65
   <211> 30
   <212> DNA
   <213> Artificial
<220>
   <223> AlstCR degenerate reverse primer
<400> 65
   atgcgcagca atgcgtagca atgcgctctt 30
<210> 66
   <211> 24
   <212> DNA
   <213> Artificial
<220>
   <223> AKHR degenerate forward primer
<400> 66
   gacctgatgt gcacgacagt catg 24
<210> 67
   <211> 24
   <212> DNA
   <213> Artificial
<220>
   <223> AKHR degenerate reverse primer
<400> 67
   gtcagatcca agtaccacag agca 24
<210> 68
   <211> 26
   <212> DNA
   <213> Artificial
<220>
   <223> Forward primer for cloning AlstAR
<400> 68
   ggaattccat ggcgtcgact gaagac 26
<210> 69
   <211> 26
   <212> DNA
   <213> Artificial
<220>
   <223> Reverse primer for cloning AlstAR
<400> 69
   cctcgaggtc agacgatgtc atggca 26
<210> 70
   <211> 28
   <212> DNA
   <213> Artificial
<220>
   <223> Forward primer for cloning AlstCR
<400> 70
   ggaattccct gcgttccatt cactgcta 28
<210> 71
   <211> 28
   <212> DNA
   <213> Artificial
<220>
   <223> Reverse primer for cloning AlstCR
<400> 71
   cctcgaggaa gaattgggtt catggcag 28
<210> 72
   <211> 29
   <212> DNA
   <213> Artificial
<220>
   <223> Forward primer for cloning DHR
<400> 72
   ggaattccga tggcggagaa gtgcctggc 29
<210> 73
   <211> 29
   <212> DNA
   <213> Artificial
<220>
   <223> Reverse primer for cloning DHR
<400> 73
   cctcgagggt cataccgtga gtcgtatgc 29
<210> 74
   <211> 30
   <212> DNA
   <213> Artificial
<220>
   <223> Forward primer for cloning AKH
<400> 74
   ggaattccgg acctgatgtg ccgagtcatg 30
<210> 75
   <211> 28
   <212> DNA
   <213> Artificial
<220>
   <223> Reverse primer for cloning AKH
<400> 75
   cctcgagggc ttgtcgatcc aataccac 28
<210> 76
   <211> 37
   <212> DNA
   <213> Artificial
<220>
   <223> Forward primer for cloning LGR1
<400> 76
   cgggatcccg gatgtattgg agattatgta tttgggc 37
<210> 77
   <211> 29
   <212> DNA
   <213> Artificial
<220>
   <223> Reverse primer for cloning LGR1
<400> 77
   cctcgagggt taaagcggta cctcactac 29
<210> 78
   <211> 31
   <212> DNA
   <213> Artificial
<220>
   <223> Forward primer for cloning or83b
<400> 78
   ggaattccga tgaccaaagt gaaggcccag g 31
<210> 79
   <211> 31
   <212> DNA
   <213> Artificial
<220>
   <223> Reverse primer for cloning or83b
<400> 79
   cctcgagggt tacttgagtt gtaccaacac c 31
<210> 80
   <211> 28
   <212> DNA
   <213> Artificial
<220>
   <223> Forward primer for cloning Oct/TyrR
<400> 80
   ggaattccgg ggcaaacagc tacacacg 28
<210> 81
   <211> 31
   <212> DNA
   <213> Artificial
<220>
   <223> Reverse primer for cloning Oct/TyrR
<400> 81
   cctcgagggt cacggtttca tacagagtaa c 31
<210> 82
   <211> 30
   <212> DNA
   <213> Artificial
<220>
   <223> Forward primer for cloning PBAN-R
<400> 82
   ggaattccga tgacattgtc agcgcccccg 30
<210> 83
   <211> 34
   <212> DNA
   <213> Artificial
<220>
   <223> Reverse primer for cloning PBAN-R
<400> 83
   taaagcggcc gctcaatcat gaatgtaaca aaaa 34
<210> 84
   <211> 21
   <212> DNA
   <213> Artificial
<220>
   <223> MiserR Forward primer 1
<400> 84
   gatttggaaa atttggacga t 21
<210> 85
   <211> 21
   <212> DNA
   <213> Artificial
<220>
   <223> MiserR Forward primer 2
<400> 85
   ggagggtctt ttgtccatgc a 21
<210> 86
   <211> 21
   <212> DNA
   <213> Artificial
<220>
   <223> MiserR Forward primer 3
<400> 86
   tctcatccaa taattgcaat t 21
<210> 87
   <211> 21
   <212> DNA
   <213> Artificial
<220>
   <223> MiserR Reverse primer 1
<400> 87
   acgtaatgct gaatatcgaa g 21
<210> 88
   <211> 21
   <212> DNA
   <213> Artificial
<220>
   <223> MiserR Reverse primer 2
<400> 88
   caaatttaaa attgaagcag t 21
<210> 89
   <211> 21
   <212> DNA
   <213> Artificial
<220>
   <223> MiserR Reverse primer 3
<400> 89
   agccaaagca agtgatataa g 21
<210> 90
   <211> 21
   <212> DNA
   <213> Artificial
<220>
   <223> MidopR Forward primer 1
<400> 90
   actcttggtg ttattatggg c 21
<210> 91
   <211> 21
   <212> DNA
   <213> Artificial
<220>
   <223> MidopR Forward primer 2
<400> 91
   tggctaggtt atgccaattc t 21
<210> 92
   <211> 21
   <212> DNA
   <213> Artificial
<220>
   <223> MidopR Forward primer 3
<400> 92
   cgagactttc gacgtgcctt t 21
<210> 93
   <211> 21
   <212> DNA
   <213> Artificial
<220>
   <223> MidopR Reverse primer 1
<400> 93
   aaaggcacgt cgaaagtctc g 21
<210> 94
   <211> 21
   <212> DNA
   <213> Artificial
<220>
   <223> MidopR Reverse primer 2
<400> 94
   agaattggca taacctagcc a 21
<210> 95
   <211> 21
   <212> DNA
   <213> Artificial
<220>
   <223> MidopR Reverse primer 3
<400> 95
   gcccataata acaccaagag t 21
<210> 96
   <211> 23
   <212> DNA
   <213> Artificial
<220>
   <223> MiserR Forward primer 1
<400> 96
   atgttagaaa atgatttgga aaa 23
<210> 97
   <211> 20
   <212> DNA
   <213> Artificial
<220>
   <223> MiserR Reverse primer 1
<400> 97
   ttattttgat gattccatca 20
<210> 98
   <211> 23
   <212> DNA
   <213> Artificial
<220>
   <223> MiDopR forward primer
<400> 98
   atgttgccct ggtggctacc tct 23
<210> 99
   <211> 20
   <212> DNA
   <213> Artificial
<220>
   <223> MiDopR reverse primer
<400> 99
   ttaaaaacat aaaaatctca 20
<210> 100
   <211> 23
   <212> DNA
   <213> Artificial
<220>
   <223> MiNprR forward primer
<400> 100
   atggaagcat ctacaatgga att 23
<210> 101
   <211> 20
   <212> DNA
   <213> Artificial
<220>
   <223> MiNprR reverse primer
<400> 101
   tcatatcctc tcatctgtat 20